# EUROPEAN PATENT APPLICATION

(11) **EP 1 873 252 A2**
(43) Date of publication of application: **02.01.2008**
(21) Application number: 07118591.2
(22) Date of filing: 26.02.2004
(51) Int. Cl.: C12N 15/82, A01H 5/00

(54) **Arabidopsis promoters**

(30) Priority: 27.02.2003 EP 03075587
(62) Divisional of application: 06121996.0
(71) Applicant: CropDesign N.V., 9052 Zwijnaarde-Gent (BE)
(72) Inventor: Hatzfeld, Yves, 59000 Lille (FR); Broekaert, Willem, 1700 Dilbeek (BE)
(74) Representative: Mistry, Meeta

(57) **Abstract**

The present invention provides several promoters isolated from *Arabidopsis thaliana,* which promoters are capable of driving and/or regulating the expression of an operably linked nucleic acid in a plant. The expression patterns of the promoters according to the present invention have been studied in *Arabidopsis thaliana* and some of the promoters displayed specific activity in particular cells, tissues or organs of the plant, while others displayed constitutive expression throughout substantially the whole plant. Some promoters showed weak expression, while others were strongly active.

SEQUENCE LISTING

<110>  CropDesign N.V.

<120>  Arabidopsis promoters

<130>  PF58316

<150>  EP 03075587.0
<151>  2003-02-27

<160>  36

<170>  PatentIn version 3.3

<210>  1
<211>  1223
<212>  DNA
<213>  Arabidopsis thaliana

<220>
<221>  misc_feature
<223>  PRO0162 - fructose bi-phosphate aldolase

<400>  1
aatactaata gaggaacaga gtggtgttga taaatgataa tgctgatgga tatgtttata      60
ggagaaaatg gaaaattatc acaaaaatag aaattgacga ttacgaagtt tctagatgta     120
ccatcttaat cgacttggag acaatttaaa tggaccatac acatccgtgt ttctatttac     180
atgtcaatat acatatattc tttgtctttt tagtatattt ccttcttttc ccctattttc     240
tttttaaata ttgtatgttc tatatcagtt tctttcttaa gatattatgg catatcgtaa     300
cagttgtttc catttataat catattttat ttttagtatg tcatagagtt ttttaaaatt     360
tatttatttg tcaacgaggt tttattaaaa aattatatac acatattaaa aaaatgttga     420
aaatacgtgt aaaaatctca taatttgtta taataataag atgtttcatt ttataatcac     480
ttgaacctaa aagataagaa acaataaaac cattgaagat cctaaaagac acctttaaaa     540
cttcaaaatg tatacaacaa caatagcaac aaaaaagttc tagactacat acatactgtg     600
tcggtagaaa gcaaaagact ttgatagttt ttgattattc atgcgtttga agagtcgcag     660
ctgttttccg gttatatgtc tctatctaaa tctaagatct taattttcta tgttcggaga     720
tatcaaagtc gcactttttc tgtgaatcta gaaacacata acatttccaa taagaatatt     780
ctattgagat tcgtagtcaa ctattaagtg tttattacga ttaaaaaact actataatca     840
atgattaatg taatttatta tcttacgatc tcaattatac aattcgtctg acggtttggg     900
ccgtcgtaag gccgaagtca tgcttttcct taaataacac tacgagttac caaattaccc     960
ctcagctaat ttgctgagaa tccacgctat taaggggtag aattaagatt agccaacatt    1020
gccaattaga gatccaacgg ctgaaaaagc tatttcttgg ggaacatgca aagatctgac    1080
ccttaattaa tattttcacc aaccaataga ctctcatccg cagctataaa accaaccctt    1140
ttcctctact ggtccaccac tcgtctgcct tcttccgcat ctcttttcat ttctctctga    1200
tttctcgatc tctccgtcca act                                            1223

<210>  2
<211>  1197
<212>  DNA
<213>  Arabidopsis thaliana

<220>
<221>  misc_feature

<223>  PRO0143 - cyclin D2

<400>  2
ggtgtttaag aatagaggaa ggatgtgaac acatgttgaa aatgatgtct aaacgatcga      60
atccaaatta acacttataa atcgatccgg aaaatcatga aaatgaaaag aaatgtatag     120
tgttagttaa actgtaatta acttaaacat gacaaaatat tttatataag tgaagttact     180
aaatgattcg tcttaataga aacatagcct tagctatata tttttttcct taggttggat     240
gtttccacaa ctccgcattt catattttgt ttctaaggtt atgtggtggt agtccgatgt     300
tttaggtcaa gttttggtcg atggttcttc tttctatctt tgacattttc ttttcttggc     360
tttcaatcag tctttcatat ctactacagc ggtaaaaaca gttgggcgtc tttaagttta     420
ttctactcta gttcaaaatc ataagtgatt gagatcttat tagttattgt tttttcggtt     480
tatatctcga cgttaccgat gttggcgaat agcacaaggt tccctaacct taacctcaaa     540
agaaagatcc actaaataga agtaaataaa gcatatgatt caatttcaga agtttacaaa     600
acactaaaca agaacatgaa aaccaaaaaa tagtgaattg tcttcttttt tgggaatagt     660
atctctggag atttcaacat ttatgaccaa cttatttttt tggaatactt ttttttatcg      720
ttgactttat gtaatttcta aattttacta tgacaatttt tttttttttt ttttttttgt      780
cgtttttact atgacaattt aaatttagtt tataaggact tgtgatatta aactattaat     840
aacaatatga tttaaatttt aaatataatt tatatttcca catattgttc ttagtgtata     900
ttctctgtat tttccatttc gagtctgtgg ggtggtctct cccctctcta ccctaaaaca     960
cactcaccca ctttcctctg tataagcctc tctctcctct ctcttctcct ctgccatgaa    1020
aatcgcagtt cctcaagaca aaacctcctc agaaatctcc catctttgat gacttttgct    1080
tccttagttt tcactttctt gtccgaacgc tctcaaaaac tttgagacca ccccaataaa    1140
cgaattaaac agactattat caatcaatac acaacacaac aatcaaacca aacccca       1197

<210>  3
<211>  1219
<212>  DNA
<213>  Arabidopsis thaliana

<220>
<221>  misc_feature
<223>  PRO0114 - cyclin D3

<400>  3
   ctcctcagcc atctgcctat ccacctccct caacctcggg ctaccctccg atcccttcag      60
   cttacccacc tcctccgcca tcctcagctt accctcctca accataccct ccgcaaccat     120
   catattaccc acaaggtacc ttcctttctt tcctgttcta tgctgcaatt gatcgcattt     180
   gcttgtctct aactatagtc agtggtggtt gatctttttt cttcttgttt ccaggtccat     240
   atccaggaca atacccacct cctccatact agagcgtttg gcgtttagaa tttgacggga     300
   agtggtcatg atgtatagat tatagttatg gtgttttcta gaatttggca gacgaatttc     360
   tttgttttgt tttttttata tcatcttcct tgtgctattg ctagcgtgat ggttaatttt     420
   tgagttggtc agtgaaactt attgagaagt ttggatcttt tcatgtttaa ccagatcgca     480
   gattatattg aattattact ctaaatcctt ggttttgtgt gttcatcatc ctcttgagtt     540
   aaaattatgt taaaaacaaa aaaaggtttt ggttctgtct ttaactttaa gaaacggaac     600
   ttatcggcgt ataatcatcg cacttgtgat cagatgcaat tagaaaaagg ttatttggtc     660
   aatgtatagc aatatcttta ggttctaaca aaaacattat tgttgacaaa tagcccaaaa     720
   gtagtagaat ttagccgaca gattttactg cactttggtt tctcgatctc tactccaaat     780
   gagtaaaaag tgggtaaagc gggaaacagc aaaagaaatc acacatttaa taaaaaataa     840
   agaaaaagaa attccacatt aaataatcac aacaagaaaa acaaataata tattagtaaa     900
   agacaaacgt aaaaaccatc tctcggcctt ctctcataaa tcaatctctc ttcttatcac     960
   tctccgaaac ccacttccag ctttttcctc tctctttctc tctctagtct ctcttttgta    1020
   gctctcccct gctaagctaa ccactgcacg tttccataga gaggaaagat gagtctctct    1080
   ccgagagatt ttctctctat catcttatct tcttccgtgt aatgctctga gccaaaaccc    1140
   aataactaaa tcaacaacaa tatagaagag aagagaaaga tcttatcttt cttctcattc    1200
   ttgagtttag tcccccaca                                                1219

<210>  4
<211>  1134
<212>  DNA
<213>  Arabidopsis thaliana

<220>
<221>  misc_feature
<223>  PRO0161 - rubisco activase

<400>  4
attgcttcgt cggcagcgaa tgaagcctaa aattaaaatt tggttttgtc cattgatgat      60
atgatggctc gttccacatg agagaatggt tgagaaaact gaaaataatg aagaggatga     120
gaaattataa tgatcagaca agtgaagtgt ttcattttat agcttagtgt agaaaacgat     180
tgcttgtgat tgtgaatata tagttttaga tgagttgttt ttatagtaac tttgagggaa     240
caataaacta tgatatagtt atcattttta ttttaaaaca agtaaattca tatgaatttc     300
tgatatattc atattttcac tatgaattta aacactataa tcttatttca tattttagca     360
tatgatattt tgcatgatgc tatcttagcc tcgactatct ttacttcatc ttctaaacta     420
tatatttatc aaatcttacg tatgcattgc atcacttgtt tttacatttt gtgtaaagaa     480
aaagttgcat taattagttt ttagcggttt agtacaatat ggttaatttg ataaccacat     540
acttgatacc tgttgtatct agagttgtat ataacaaaat tagaattata ttctattggc     600
taagccaatt tctctccacc aatcagttta catcggttat ttggaacatt attatcgata     660
agcaatgtca ttatccaaaa ttttggaatg ttatcgtatg aagaagagtg tagaagtatc     720
ttgatcacat atgaatggct tacctcaatc ctcaaaaaaa aaaaaaaaca atgattttcc     780
aaagcatatg tggtaataaa taatgttata atcttcactt gaaaaaaaaa acaaatccat     840
tggcacagat tagccatttg acgacaacct cataaacata ttccacgtgg accaatcaaa     900
atagaatcct cctaatcatc ttcctcgtgg caacttggcc aatccgcatc gtgtggcgat     960
cagagagtta agccttgaag acgaagagat aacgaattgg ttgttgatca ctcgctttat    1020
aaatctctca gtttcttgct cacaccaaca tctctctaag cttcttcttc taccaatcta    1080
attcctctct tcagcttctt gtgttgtgac gcatactcgt cgcagtcttg agat          1134

<210>  5
<211>  1239
<212>  DNA
<213>  Arabidopsis thaliana

<220>
<221>  misc_feature
<223>  PRO0183 - putative extensin

<400>  5
cacatgcact ctatagtttt atgtcgtcat gatatacata cattgggaaa ttatacgacc      60
aatacgagta aacgaaataa aaaatgaatg gtcaattata tgacacaatt caaattctaa     120
acggaactaa attttagcct ctatggttat gtatactatt attacagtcg ataagtgtga     180
agcgagtcga cgcggtatat catacaccga tcgagccaca acacgcgacc tctcattata     240
ttaaaatcac atttttatgc cacaaatcaa agtcgttgtt gttaatttgg tttaattgaa     300
ctttgtatta taactcggtt tagtcttcaa atacagctgt cttcctattg atcgctttgt     360
tatcttatgt cagccatttt aagacatttg actttactct cataatcatc cttttctgta     420
catttatttg attcttacgg ttctaattac ctttctatat atatgatatg tttttgtcaa     480
aagcatcaag tgtttttgta gtttatattt gtagtttcta agtttatgac tttggaaaaa     540
ttaggaacgt tctggcacca cattgtaata tgcatgatcg gaaattttgt ggtcctcttg     600
ccataatttt cgcgcaccaa caagtgtttt tatagatttc gtgtaatcgt ttatttttga     660
aatattttat agctttctgt tgactgtata tttgtgattt atacgcaatt ctttaacata     720

tacagaaaca gaatgtcggt aaataaaaaa gaaattatat aatcgaaaat gattgtggta    780
gatgtcatcg tgattaacat agcttaacta gcatcgaata gcagcatgta atttaaaaat    840
gtttcattga aatatatgtg tgattttcaa tcagaattag tttggcttta tttcaatctg    900
gtggttggag attagttttc tcacacagtg gaaatttcct gatctattat cttgtttatt    960
ataaaactaa gaaaaccaat aattgccagc taatagcaaa attaaaaaaa aactatagga   1020
aaaaaataac gaagatatag aattagtccc taaagaacaa agcccacctc caacgactac   1080
ttttttcact ataaaaacaa ctcttttcca tgcagaaaca caatcaatca cttcttcttc   1140
atctttcctc aaaaataaaa acattttctc caatttgttc ttctttaata atttggagaa   1200
aaaaagtctt agctaagact gaacattatc cggtgaaga                          1239

<210>  6
<211>  1067
<212>  DNA
<213>  Arabidopsis thaliana

<220>
<221>  misc_feature
<223>  PRO0185 - 12S cruciferin AtCRU3

<400>  6
cagattcagc acacaaagcc agtaaagata gaaaatttaa cgaacgctca tgctaagctg     60
cgcaaaatac ttcctaatca aaacagtaac aacgagtaat tagcaaaatc cgagcagaaa    120
actctcaccc acctccgaaa ttcacgtctt cactaaaatt ttcgaaagga atcgatcaat    180
accaacccat tacacaaaat acataatcaa aatggcgaga atcgtacctg gaaactttgc    240
ttcaagtcgc agagagagga aaaggaagat cgtggagaaa ggggtttagg gtttaagctc    300
agacttctat tggagtaaat gggacggtgt cacattttcc gttttggaaa tgaactttgg    360
gctcacgtta tgggctatta gatatttgat gggctttcta gtaaatacaa tataagttat    420
tgggcttagt ttaaataagc ccatgttgga aatatttgac acatgtcttg gctactagtg    480
ctaaacatgc aaccgaacag ttgtcgagac aagtcgcagc atatacaatg gatcaaacac    540
gcctagtgtc gccgcgtctc gctcatgtgt caccttgttt cctcgttttt ttttaatttt    600
tcataagttc ttttgtttta tcttcaatac aaatttttgg ctgtatcttg caaactcttc    660
gatcatatcg ccaatatacg tgaacactgg tgatctaatt tgttgtgtta attgttaaat    720
ttagattcta ttctccggtt taaaagtgaa ttatatgtat catggttaaa acattgtaag    780
taagatgata ataaaatgat aaatttagtt gatggataac gtgaagcaaa aaatgagata    840
gatacatttg attttgtcgt attttgacat atgcggagag tgagctacgc gcatgaagat    900
caagagacac ttgctcgagc tcacagagtg acgtgtaaaa agcttagact gaagtcccca    960
tgcaaaccta atcctacgtg gctcaaacca cgagctcact tgacaatata taaactcctc   1020
ctaagtcccg ttctcttcat ccatctctca caacaaacaa aaagaaa                 1067

<210>  7
<211>  1178
<212>  DNA
<213>  Arabidopsis thaliana

<220>
<221>  misc_feature
<223>  PRO0190 - putative protein

<400>  7
tccagataca tcatgggctt tgggatgatt tagaattagt tagtaaacaa aacaaacaat     60
tatgtttaat cgagatgtta tcttctggtc acaaattaag ccttcattga ctaaattaaa    120
gtccactcca agattaggcc catgagcatc agaaacgcag tccattccaa gataaggccc    180
actagtttct catgctagtc tgttaacgca gatttaaaag aaatatgtac gatacaaaat    240
gtgtctgaaa atcaattata aatttataat aatgtactta taaaaacgta tataaaactg    300

taatgatttc tgtttatatt ttaatattat tcaaaaatgt tgtacaaata cgaccaagaa 360
tattaatagg tgttaatcat atactatacg atcataatat ttaccctcaa tctaaaaggt 420
aaaaaaacct cttgaatcat caggaccaag tacagttcat tgaccatgcg aaagcataaa 480
ttatattttt ccaatgaaat atttttcatt caggaaaaga ctctagtttt caatttcatg 540
tcgaaatgct gtcaaacaca tttttcttag atagataaac taaatataag tttgatttga 600
gttagtaagt tagatacatt catacataga ttggttgtta taaatgacca cactcttgat 660
tttactttct tgacaggtgg ttgtaatttc attgaccaca agttataatg cagatatcgc 720
ttaacttatt aaggtacggt atcgatattt tcatttagtc accatcataa gttaccacgt 780
cttcttcttc ttttgcctta tcttccatac acctacttgg atcggttgag agtaaacata 840
taacataaca aggagtcaag ggcatatagt aacgtgtgat aataatatat atttcactga 900
atcaggtata tcatgttaat taccttacgt aatgacaaat gtcaaatgtt gtatggtagt 960
tagcaatata gaaaaacttg ttaatattcc attgatacct aaataagcta tcaacttagt 1020
attcacgtaa tgtcctaaac aattcaattc taaccaacat tataacttaa tatcgttgaa 1080
ttgttagacg aaaataaaat ttgaacccta tataaattgc taagttcttg cgtacatgct 1140
ttgtagacat aaattattag agagacgaaa ggaaaagc 1178

<210> 8
<211> 2510
<212> DNA
<213> Arabidopsis thaliana

<220>
<221> misc_feature
<223> PRO0193 - FAD2 desaturase

<400> 8
aaacagtggt taggattgta tgacgccttt tattcatatt cttgttatct ccgttatgtc 60
atgtgtgtga atcacttata taattttcgt aagattttct gaatatgttg gagtctttgc 120
taactgtttg aatcgagatc agttaacact tattaagaac aaaaatgtgg tttcttgtga 180
gaaaaatggt ttaataaaaa tccgtgattg atagaagaaa aagatcaaaa taaatggttg 240
gtgacgggtg atcttaaaaa tgttgaaatt aaggtgtgtc gtcgttatac gcggtaaata 300
gatagataga aaaatagaag tccaatgcaa gagacttaac ttaatcatcc caattaattg 360
attgcattaa cttgtacttg tattttccgt ccgccaccta atttgattaa taatataata 420
aagattacaa ttgaaaacat aaacaagaga aaatccgcac gaatctacca aagtgcatca 480
cgtttgggta tccatacacg tgaccaccag tccaccacaa cacaatgtct gtagatattt 540
taatgtttca catgatagaa gaagccaaac gtaagaactc tcttttccac ttttagccct 600
ttccccgcct accactgctt acgacttgtg taagtggcaa actagtaata atagagacga 660
aacttaaata taaaaaagtt gaatccaacc aagttggtgt taatcaaatg gttaagttat 720
aatggtgaaa gatttgccat gtgtattgta ttaagagtta agaccaaggt ttggttccca 780
tcacttacga ttctttcttt tcatatgatt ctaaagttag ttattataaa catcttaatt 840
tactacacaa tattcggtaa tttctacata ttttagagat tagtttgagt ttcaatccat 900
actttactag tgattataaa ttaatatacg tacttttcga ctataaagtg aaactaagta 960
aattagaacg tgatattaaa aagttaatgt tcactgttat atttttttca caagtaaaaa 1020
atgggttatt tgcggtaaat aaaaatacca gatattttga attgattaaa aaggttgaaa 1080
taagagagga ggggaaagaa aagaaggtgg gggcccagta tgaaagggaa aggtgtcatc 1140
aaatcatctc tctctctctc tctctacctt cgacccacgg gccgtgtcca tttaaagccc 1200
tgtctcttgc cattccccat ctgaccacca gaagaagagc cacacactca caaattaaaa 1260
agagagagag agagagagag acagagagag agagagattc tgcggaggag cttcttcttc 1320
gtagggtgtt catcgttatt aacgttatcg cccctacgtc agctccatct ccaggtccgt 1380
cgcttctctt ccatttcttc tcattttcga ttttgattct tatttctttc cagtagctcc 1440
tgctctgtga atttctccgc tcacgataga tctgcttata ctccttacat tcaaccttag 1500
atctggtctc gattctctgt ttctctgttt ttttcttttg gtcgagaatc tgatgtttgt 1560
ttatgttctg tcaccattaa taataatgaa ctctctcatt catacaatga ttagtttctc 1620
tcgtctacaa aacgatatgt tgcattttca cttttcttct ttttttctaa gatgatttgc 1680

tttgaccaat ttgtttagat ctttatttta ttttattttc tggtgggttg gtggaaattg 1740
aaaaaaaaaa aaaacagcat aaattgttat ttgttaatgt attcattttt tggctatttg 1800
ttctgggtaa aaatctgctt ctactattga atctttcctg gattttttac tcctattggg 1860
tttttatagt aaaaatacat aataaaagga aaacaaaagt tttatagatt ctcttaaacc 1920
ccttacgata aaagttggaa tcaaaataat tcaggatcag atgctctttg attgattcag 1980
atgcgattac agttgcatgg caaattttct agatccgtcg tcacatttta ttttctgttt 2040
aaatatctaa atctgatata tgatgtcgac aaattctggt ggcttataca tcacttcaac 2100
tgttttcttt tggctttgtt tgtcaacttg gttttcaata cgatttgtga tttcgatcgc 2160
tgaattttta atacaagcaa actgatgtta accacaagca agagatgtga cctgccttat 2220
taacatcgta ttacttacta ctagtcgtat tctcaacgca atcgtttttg tatttctcac 2280
attatgccgc ttctctactc tttattcctt ttggtccacg cattttctat ttgtggcaat 2340
ccctttcaca acctgatttc ccactttgga tcatttgtct gaagactctc ttgaatcgtt 2400
accacttgtt tcttgtgcat gctctgtttt ttagaattaa tgataaaact attccatagt 2460
cttgagtttt cagcttgttg attcttttgc ttttggtttt ctgcagaaac 2510

<210> 9
<211> 1050
<212> DNA
<213> Arabidopsis thaliana

<220>
<221> misc_feature
<223> PRO0194 - G3PDH-like

<400> 9
gaggaggaga agaagaagac ttttggcaaa agccaagagt catacctatt agtctattac 60
ataaggtgtg atgttttttt ttgctgtcac attccatctg acctaccaaa gctctcaaag 120
tggagcatct attgtgtctt ggtatagagt cacgattaga taaatataca acaccatatg 180
tacttgtgtc aaattataaa tatttgaaac agtaagcaac cagtaattcg ataaagacgt 240
ctacaagcat ctacacatct aaattgatta aaacaaaaca cctttaaata tagtgtttat 300
gaagtcaaat cttcaccttg attattctaa aacaatggtt ggagtaatgt tgctgaatgt 360
tgagtgagaa ttgacatttg agagtaactt tcactataat gaactggatg ataaagttga 420
tccgataata attgaattta ccatgaatgg tatcacaaga tacatgtata gaagacagtg 480
gtgttacttg ttacgcaaaa ttaaaaaatg agcatactat tgcagttact ttggatttat 540
taaggaaaat tatggtttga caacaacaat aatacaaaat cttatgaaaa ttaaataaaa 600
agaaaaacaa atttggctat tggcaaagct cattggctgt caaaaggaat atatacaaaa 660
tctgctacgt tgcagtcttg cgtgcaccgg cccagcccat aggattagag tattaaacct 720
cgaatattcc atcagcctgc gcgtgaatcc aagcgccatt agtttcccca aatcagttct 780
taatcctacc cataaacgat gggtaaaaat ggtaaataag aaagaaagta aagtacaata 840
tagtaatatt aattagtgaa tctaatctat tagccttttt cccaagaaaa aatctcagcc 900
actcgatcat attttcaatt ttcatcatca cgttcttctt ctcttttaaa taaccctaaa 960
tcctcaccaa acccaaaccc tcactcacta ttttcacatt ctcttctctc tcgatatcat 1020
ctaaatctct ctcgatctca atttcgcaaa 1050

<210> 10
<211> 1781
<212> DNA
<213> Arabidopsis thaliana

<220>
<221> misc_feature
<223> TC149436 (PRO0162)

<400> 10

gaccactcgt ctgccttctt ccgcatctct tttcatttct ctctgatttc tcgatctctc 60
cgtccaacta tgtctgcctt cacaagcaaa ttcgccgatg agttgatcgc caacgctgcc 120
tacatcggca cacctggaaa aggtattttg gctgctgatg agtccactgg taccattgga 180
aagcgtcttg cgagcatcaa cgtcgagaac gttgagacca acagacgtaa cctccgtgag 240
cttctcttca ccgcccctgg tgctcttcca tgcctcagtg gtgtcatcct tttcgaagag 300
actctgtacc aaaagagttc cgatggtaag cttttcgttg atatcttgaa ggaaggagga 360
gttcttcccg gtatcaaggt tgacaagggt accgttgagc tagctggaac cgacggtgag 420
accaccactc aaggtcttga cggtctcggt gacagatgca agaagtacta cgaagctggt 480
gctcgtttcg ccaagtggcg tgcagtcctc aagatcggag agaacgagcc atctgagcat 540
tccattcatg agaacgctta cggattagct agatacgctg ttatctgcca agagaacggt 600
cttgtaccaa ttgtcgagcc tgagatccta gtcgatggat cccatgacat ccagaagtgt 660
gctgccgtga ctgagcgtgt ccttgcagct tgctacaagg ctcttagcga ccaccacgtc 720
ttgctcgagg gtacactctt gaagcctaac atggttactc ccggatctga cagccccaag 780
gtttcacctg aagtcatcgc tgagcacacc gtccgtgccc ttcagagaac cgtcccagca 840
gctgttccag ccattgtctt cttatctgga ggacagagcg aggaagaagc taccaggaac 900
ttgaacgcca tgaaccagtt gaagaccaag aagccatggt cattgtcttt ctcattcgga 960
cgtgcgttgc agcagtctac cttgaagaca tgggcaggta aagaggagaa tgtcaaggca 1020
gctcaagagg cgttgtatgt gaggtgcaag gctaactctg aagccacact cggaacctac 1080
aagggtgacg ctaagcttgg tgatggagca gctgagagcc ttcacgtgaa ggattacaag 1140
tactgagcat cgtccacggg ttgaattgtg acgtttgtca tcttcctgga tatatttcta 1200
gggatttttc gtttttctac ttgacattat cggattataa gtgttttttc ttcattttcc 1260
ttttggattt gtgttttgat tttgattggg ttttgttttt tagaattgag tattttaaaa 1320
acacgcccaa ataaaaaagt catttgaggt tatttctgtt gaacctcttg tggaatttga 1380
aaatcagttt ctgttgattt accgcttctt gagcctaaat tattgtattt ggttctctga 1440
ttcgtatatg aaatctcgtc ttttaaacac ataaaaatct gtctatgaag agtatgaacc 1500
aagcgttcct gtttcatttt ttcaacaata tgactagtat gtgagagcat aattgctata 1560
tgaggctaca accataagct gaaaggagcc tcgaacaaga ttcagaaaca taggaaagtt 1620
atacactttc tcaattaatt tgcagacaca agaatatgaa caagactgta gtgtgttttc 1680
attgtcttca aaagcattcc aaaagccagc ttattgccaa atgctgtaat gagaatacac 1740
agctaattta tgatattctg tttatgtgta agatgggcct c 1781

<210> 11
<211> 1723
<212> DNA
<213> Arabidopsis thaliana

<220>
<221> misc_feature
<223> TC156804 (PRO0143)

<400> 11
gggtggtctc tcccctctct accctaaaac acactcaccc actttcctct gtataagcct 60
ctctctcctc tctcttctcc tctgccatga aaatcgcagt tcctcaagac aaaacctcct 120
cagaaatctc ccatctttga tgacttttgc ttccttagtt ttcactttct tgtccgaacg 180
ctctcaaaaa ctttgagacc accccaataa acgaattaaa cagactatta tcaatcaata 240
cacaacacaa caatcaaacc aaaccccatg gctgagaatc ttgcttgtgg tgaaaccagc 300
gagtcatgga tcattgacaa cgacgatgat gatatcaact atggcggcgg atttacgaac 360
gagattgatt acaatcacca actttttgct aaagacgaca actttggcgg caacggatca 420
attccgatga tgggttcttc ttcatcgtcc ttgagtgaag acagaatcaa agagatgttg 480
gtgagagaga ttgagttttg ccctggaact gattatgtta agagattgct ttctggtgat 540
ttggatttgt ctgttcgaaa ccaagctctt gattggattc taaaggtttg tgctcattac 600
cattttggac atctgtgcat atgcctatcc atgaactact tggatcggtt cttaacatcc 660
tatgaattgc cgaaagacaa ggattgggct gctcagttac tagctgtgtc ttgcttatca 720
ttagcatcca aaatggaaga aactgatgtg cctcacattg ttgatttaca ggtggaagat 780

cccaagtttg tttttgaggc caaaacaata aaaaggatgg agcttttggt tgtcaccact 840
ttgaattgga gattgcaagc tctaactcca ttctccttca ttgattattt cgttgacaag 900
atcagtggtc acgtgtcgga gaatttgatc tatagatcgt caagattcat cttaaacacc 960
accaaagcaa ttgaattctt agacttcagg ccttctgaga tagctgcagc tgctgcagtg 1020
tctgtttcca tttcaggaga aacagaatgc attgatgagg aaaaggcact gtctagtctc 1080
atatatgtaa aacaggagag ggtgaagaga tgtttgaatc tgatgagaag tctcactggg 1140
gaggagaatg tgcggggaac tagtttatcg caggagcagg cgcgagttgc ggtaagagct 1200
gtacctgcaa gtccagttgg agtgttggaa gcaacatgtt tgagctatag gagtgaagag 1260
agaacagttg agtcatgtac aaattcctca cagagtagtc cagacaacaa caacaacaac 1320
aacaacagca acaagaggag gagaaaacaa tgagagagaa taaaagagtc atacattgct 1380
ttttacaacc caaaaccaca agtactcatg acatttgagg ttcttattta tttttttggt 1440
tttttttttc tacataaatt ttctttttct ttctttgatt tctcattttc aatctgaaaa 1500
ttggattgaa tatgagagtt ttgtgagaaa ggaaaaaaga aaataagaga gagagagaga 1560
gctctttgga aggcgggcaa aattaataag tcattattga tgatgatgag agacatccct 1620
gttcttgctc caagggactt ttttttttct acataatgtc agagatataa ttaaaaaaaa 1680
aagaaataga aagagaatta attttatgaa aaaaaaaaaa aaa 1723

<210> 12
<211> 1647
<212> DNA
<213> Arabidopsis thaliana

<220>
<221> misc_feature
<223> TC163888 (PRO0144)

<400> 12
atcactctcc gaaacccact tccagctttt tcctctctct ttctctctct agtctctctt 60
ttgtagctct cccctgctaa gctaaccact gcacgtttcc atagagagga aagatgagtc 120
tctctccgag agattttctc tctatcatct tatcttcttc cgtgtaatgc tctgagccaa 180
aacccaataa ctaaatcaac aacaatatag aagagaagag aaagatctta tctttcttct 240
cattcttgag tttagtcccc cacaatggcg attcggaagg aggaagaaag tagagaagaa 300
cagagcaatt cgtttcttct tgatgctctc tactgcgaag aagagaaatg ggacgatgaa 360
ggagaagaag ttgaagaaaa ctcttccttg tcttcttctt cttctccatt cgttgttttg 420
caacaagatt tgttctggga agatgaagat ctggttacac tcttctccaa agaagaagaa 480
caaggactca gctgtctcga tgatgtttat ctttccacgg atcgaaaaga agctgttggt 540
tggattctga gagtcaacgc tcattatggc ttctctactt tagcagctgt tttagccata 600
acttatctcg ataagttcat ctgtagctac agcttacaga gagacaaacc atggatgctt 660
cagctcgttt ctgtcgcgtg tctctcatta gctgctaaag tcgaagaaac ccaagtccct 720
cttcttctag actttcaagt ggaggagaca aagtatgtgt ttgaagcaaa aaccatacag 780
agaatggagc tactgattct gtctactctc gagtggaaga tgcatctcat tactccaatt 840
tcgttcgtag accacattat caggagattg ggacttaaga acaatgctca ctgggatttc 900
ctcaacaaat gccaccgtct cctcctctct gtaatctccg attcaagatt tgtcgggtac 960
ctcccatcag tagttgccgc agctaccatg atgcgaatta tagagcaagt tgatcccttt 1020
gaccctcttt cataccaaac taatctcctc ggtgtcctta acttaaccaa ggaaaaggtg 1080
aaaacttgct acgatctaat cctccaacta ccagtggacc gcatcggttt acagatccaa 1140
atccaatctt ccaagaaacg caagagtcac gattcatcat catcgttgaa cagtccaagc 1200
tgcgtgattg atgcaaaccc tttcaatagc gacgaaagct caaacgattc gtggtcagcg 1260
agttcgtgca acccaccaac gtcgtcgtcg tccccgcagc aacaacctcc attgaagaag 1320
atgagaggag ctgaagagaa tgagaagaag aagccgattt tgcatctgcc atgggcaatc 1380
gtagccactc cataatcgaa agctcgattt cgtttatatg atatttactg tttttttaaa 1440
ctttgagaac aatctttgtt gtattaagct ttacccgttt gcatatacga aatgtcgcga 1500
atgcccttac gtgccatggc ttgatagagt taatgggtaa agggtattca tgacatttga 1560
ctgcatggga tgtgacgaag gagagaatta gaaataataa taataatatt gcgtaaattt 1620

tgaggcttgc ccaatgcttt gggccgt 1647

<210> 13
<211> 1694
<212> DNA
<213> Arabidopsis thaliana

<220>
<221> misc_feature
<223> TC160636 (PRO0161)

<400> 13
aagcttcttc ttctaccaat ctaattcctc tcttcagctt cttgtgttgt gacgcatact 60
cgtcgcagtc ttgagatatg gccgccgcag tttccaccgt cggtgccatc aacagagctc 120
cgttgagctt gaacgggtca ggatcaggag ctgtatcagc cccagcttca accttcttgg 180
gaaagaaagt tgtaactgtg tcgagattcg cacagagcaa caagaagagc aacggatcat 240
tcaaggtgtt ggctgtgaaa gaagacaaac aaaccgatgg agacagatgg agaggtcttg 300
cctacgacac ttctgatgat caacaagaca tcaccagagg caagggtatg gttgactctg 360
tcttccaagc tcctatggga accggaactc accacgctgt ccttagctca tacgaatacg 420
ttagccaagg ccttaggcag tacaacttgg acaacatgat ggatgggttt tacattgctc 480
ctgctttcat ggacaagctt gttgttcaca tcaccaagaa cttcttgact ctgcctaaca 540
tcaaggttcc acttattttg ggtatatggg gaggcaaagg tcaaggtaaa tccttccagt 600
gtgagcttgt catggccaag atgggtatca acccaatcat gatgagtgct ggagagcttg 660
agagtggaaa cgcaggagaa cccgcaaagc ttatccgtca gaggtaccgt gaggcagctg 720
acttgatcaa gaagggaaag atgtgttgtc tcttcatcaa cgatcttgac gctggtgcgg 780
gtcgtatggg tggtactact cagtacactg tcaacaacca gatggttaac gcaacactca 840
tgaacattgc tgataaccca accaacgtcc agcttccagg aatgtacaac aaggaagaga 900
acgcacgtgt ccccatcatt tgcactggta acgatttctc caccctatac gctcctctca 960
tccgtgatgg acgtatggag aagttctact gggccccgac ccgtgaagac cgtatcggtg 1020
tctgcaaggg tatcttcaga actgacaaga tcaaggacga agacattgtc acacttgttg 1080
atcagttccc tggtcaatct atcgatttct tcggtgcttt gagggcgaga gtgtacgatg 1140
atgaagtgag gaagttcgtt gagagccttg gagttgagaa gatcggaaag aggctggtta 1200
actcaaggga aggacctccc gtgttcgagc aacccgagat gacttatgag aagcttatgg 1260
aatacggaaa catgcttgtg atggaacaag agaatgtcaa gagagtccaa cttgccgaga 1320
cctacctcag ccaggctgct ttgggagacg caaacgctga cgccatcggc cgcggaactt 1380
tttacggtaa aacagaggaa aaggagccca gcaagtaaac ctgccagttc ctgaagggtg 1440
tactgatcct gtggctgaaa actttgatcc aacggctaga agtgacgatg gaacctgtgt 1500
ctacaacttt tgagcaatat tatcctgctt attaatttgc tgttttactc ctattgtctc 1560
ttttggttta tttttctcct ttgtgtaatt gtggattgga tcttgtcctc ttttgttccc 1620
tttttttttt tttatgatgt acaacacatt ggtaatttaa aattgccttg tcataaacta 1680
cacttcttat tcct 1694

<210> 14
<211> 1354
<212> DNA
<213> Arabidopsis thaliana

<220>
<221> misc_feature
<223> TC160873 (PRO0183)

<400> 14
   tacaccgtac tattatcact ctcctccccc accggtgaaa tccccaccgc caccgtacta 60
   ttatcactct cctcccccat actactacca ttcaccgccc cctcccgtaa agtctcctcc 120
   tcctccatac tactatcact ccccacctcc tccggtgaag tccccaccac caccatacta 180
   ctaccattca cctcctcctc cggtgaagtc tcctcctcct ccatactact accattcacc 240
   gcctcctccc gtaaagtctc ctcctcctcc atactactac cactccccac ctcctccggt 300
   gaagtcccca ccaccaccat actactacca ttcacctcct cctccggtga agtctcctcc 360
   tcctccatac tattaccatt cacctcctcc tccggtgaag tctcctcctc ctccatatta 420
   ctaccattca ccacctcctc cggtgaaatc cccaccacca ccatattact accattcacc 480
   acctcctccg gtgaaatccc caccaccacc atactactac cattcacctc ctcctcctgt 540
   gaaatctcct cctcctccat attattacca ctccccacct cctccggtga aatccccacc 600
   accaccatac ctttacagct ctccaccacc accggtaaaa tctcccccac ctcctgtcta 660
   catctacgct tctcccccac ctcccacaca ttactaggct catcaaattc aacaaaattt 720
   ccccattatt caagttttct tttaaaaaat gaaataaagg aagggtcaaa gtcaaagaga 780
   agaagaagtg aaccaaataa ggatcgtggc gtggaaatca gaaacaaaac atctctaaca 840
   acgatccaca tcacttcctc ttcttcaatg tcgctcatct tccacttgtg catcattatc 900
   tttgcgtctt cgtcaagtca aattgtttcc aagacccttt tagatcgtat catactcctc 960
   tttgttccct tttgttgtta agtgaaaaaa tgggttttgg taatttaatt gttgtgcata 1020
   cattttctat tttgttattc ttccgttaat gtaatgaaag agttgttagc atataaaaca 1080
   aagttcagcc taactccatc tactatgtcg cctgatcaaa accaaacaca aaactttgat 1140
   ttagcttcaa acaaaaagaa gaatccacga ataagagaga gctgttttga atctgtttct 1200
   tgtttatttt cccctttttc gtgtcatatt atattgagag acttatgcat ccaaaagaac 1260
   aaacaaatgg ttatatgtaa cagagagaaa tcaaatcaaa atcacacatt atcaaccaaa 1320
   ggtttacagt accaactaag caagtgtgga atga 1354

<210> 15
<211> 1784
<212> DNA
<213> Arabidopsis thaliana

<220>
<221> misc_feature
<223> TC160506 (PRO0185)

<400> 15
gatccatctc tcacaacaaa caaaaagaaa atggttaagc tcagcaatct cctcgttgca 60
accttcgggg ttctcctcgt ccttaacggc tgccttgcga ggcagtcact tggggttcct 120
cctcagctac agaacgagtg taacctcgac aacctagatg ttctccaagc caccgaaact 180
atcaagagtg aagccggtca gatcgagtac tgggaccaca accaccctca gctccgatgt 240
gttggtgttt ccgttgctcg ttatgtaatt gaacaaggcg gtctttactt gcccaccttc 300
ttcacttccc caaaaatttc ctacgtcgtt caaggaacgg gtatcagcgg aagagtggtc 360
cctggatgtg ccgagacctt catggactcg cagccgatgc aaggacaaca acaaggccaa 420
ccatggcaag gacgacaggg acaacaaggc caaccatggg aaggacaggg acaacaggga 480
caacaaggaa gacaaggcca accatgggaa ggacagggac aacagggaca acaaggacga 540
cagggacaac aaggccaacc atgggaagga cagggacagc agggacaaca agggttccgt 600
gacatgcacc agaaggtgga acatgtgaga cgcggagacg tctttgccaa cactccaggc 660
tctgcccact ggatctacaa ctcaggagaa cagccacttg tcatcatcgc tcttctcgac 720
atcgccaact accaaaacca actcgaccgc aaccctagag tgttccattt ggccggaaac 780
aaccagcagg gaggctttgg cggttcacag caacaacaag aacagaaaaa cttgtggagc 840
gggttcgacg cacaggtcat agctcaagca ttgaaaattg acgttcagtt ggctcagcag 900
cttcagaacc aacaagacag cagaggaaac atcgttcgtg ttaagggacc tttccaggtc 960
gtgaggccac ctctaagaca gccctacgag agcgaggagt ggagacaccc acgtagccca 1020
cagggcaacg gccttgagga gactatctgc agcatgaggt cccacgagaa cattgacgac 1080
cctgctcgtg ctgacgtgta caagcccagc ctaggtcgcg tgaccagcgt caacagctat 1140
accttgccca tcttggagta tgtcaggctc agtgccactc gtggcgttct ccagggtaat 1200
gcgatggtgc ttcctaaata caacatgaac gctaacgaga tcttgtactg cactggagga 1260
caaggaagga tccaagtggt caacgacaac ggacagaacg tgttggacca acaggtgcag 1320

aagggacagc tcgtggtcat cccacaaggg ttcgcatacg ttgtccagtc ccacggaaac 1380
aagttcgagt ggatctcttt caaaactaat gaaaacgcaa tgatcagcac tttggcgggt 1440
agaacctcgc tcttgagggc attgccattg gaggtcatat caaatggttt ccagatctct 1500
cccgaggaag ctaggaagat caagttcaac acacttgaga ccactttgac ccgcgctgcc 1560
ggtaggcaac aacaacagtt gatcgaggag attgtcgagg cttaaatcaa aacgtttttc 1620
tttttcttaa taaagtatgg tcagtttgta atcacgtccc tttaccttta acgtacgtgt 1680
aaaatatgtg tctgcggcac ctcacttgta ataacacttt cttctcataa ataaaaggga 1740
agtttcgagt tacatactat aatatagcgc cagttttttc gtct 1784

<210> 16
<211> 1138
<212> DNA
<213> Arabidopsis thaliana

<220>
<221> misc_feature
<223> TC149672 (PRO0190)

<400> 16
gagacataaa ttattagaga gacgaaagga aaagcatgtt taaactctgt ctcgttctcg 60
tcttggttat tgcagtagat gcaaatccgg ctaagtccgg ttcttgtagt tgctccggga 120
atatatcagc gagcgatgtc gatcgagttc atttcgcgat gaacttggag ttcaccgagg 180
ccgagttctt cttaaaaggg gctaccggaa aaggacttga tgcctataac gcgacacttg 240
ctaaaggcgg accaccgccg ataggggcca agaaagccaa ccttgatcct ataactaatc 300
gaatcatcga ggagtttggt tatcaagaaa tcggtcatct tagggcgatt acggatatga 360
cgggaggaat tccacgtccg ctaataaatc tgacgagaga gaatttcgca gtgtttatgg 420
atagagcggt tggacgcaaa tctaatccgc ggtttgatcc ttacgccaac tcactcaact 480
atctcttagc ttcatattat atcccttatg ttggccttac cggttatgtc ggaaccatcc 540
cttaccttgt ctacttcaat atcaaaaagc tagtggcggg tctattgggg gtggaatcgg 600
gtcaagacgc agtgataaga acgcttctgt acgagagaca gaatgagaag gtggaggaat 660
acggtggagt cacggtggct gagctgacca atgagatatc taacctccgt aatgaactcg 720
gtatgtgcgg gatcaaggac gaaggcttgt gcgtgccgtt gtggctagga gcggagaatc 780
gcaccaccag caacatcctc tccgctgatc cttactctct ctcttatgac cggacagcac 840
aagagatcct cagggttatg tacggcaccg gtgatgagca ccgacctggc gggttttggc 900
cgtgtggtgc caatggaagg atcgctagga tgtttcttga tgaagggtgt tatggtgaat 960
attgcgttgt atgttcacat gacaattaaa agtttccatt gatacaattc actagaaaat 1020
aaaatcgtga ttaagattgt aatagtgatc tcattaattt aatactggtg attattagtc 1080
catgtattta ttgctatcga agtttaataa ataaataaaa ccgtgttgcc atttttgt 1138

<210> 17
<211> 1617
<212> DNA
<213> Arabidopsis thaliana

<220>
<221> misc_feature
<223> TC160765 (PRO0193)

<400> 17
ggaccaccag aagaagagcc acacactcac aaattaaaaa gagagagaga gagagagaga 60
cagagagaga gagagattct gcggaggagc ttcttcttcg tagggtgttc atcgttatta 120
acgttatcgc ccctacgtca gctccatctc cagaaacatg ggtgcaggtg gaagaatgcc 180
ggttcctact tcttccaaga aatcggaaac cgacaccaca aagcgtgtgc cgtgcgagaa 240
accgcctttc tcggtgggag atctgaagaa agcaatcccg ccgcattgtt tcaaacgctc 300

aatccctcgc tctttctcct accttatcag tgacatcatt atagcctcat gcttctacta 360
cgtcgccacc aattacttct ctctcctccc tcagcctctc tcttacttgg cttggccact 420
ctattgggcc tgtcaaggct gtgtcctaac tggtatctgg gtcatagccc acgaatgcgg 480
tcaccacgca ttcagcgact accaatggct ggatgacaca gttggtctta tcttccattc 540
cttcctcctc gtcccttact tctcctggaa gtatagtcat cgccgtcacc attccaacac 600
tggatccctc gaaagagatg aagtatttgt cccaaagcag aaatcagcaa tcaagtggta 660
cgggaaatac ctcaacaacc ctcttggacg catcatgatg ttaaccgtcc agtttgtcct 720
cgggtggccc ttgtacttag cctttaacgt ctctggcaga ccgtatgacg ggttcgcttg 780
ccatttcttc cccaacgctc ccatctacaa tgaccgagaa cgcctccaga tatacctctc 840
tgatgcgggt attctagccg tctgttttgg tctttaccgt tacgctgctg cacaagggat 900
ggcctcgatg atctgcctct acggagtacc gcttctgata gtgaatgcgt tcctcgtctt 960
gatcacttac ttgcagcaca ctcatccctc gttgcctcac tacgattcat cagagtggga 1020
ctggctcagg ggagctttgg ctaccgtaga cagagactac ggaatcttga acaaggtgtt 1080
ccacaacatt acagacacac acgtggctca tcacctgttc tcgacaatgc cgcattataa 1140
cgcaatggaa gctacaaagg cgataaagcc aattctggga gactattacc agttcgatgg 1200
aacaccgtgg tatgtagcga tgtataggga ggcaaaggag tgtatctatg tagaaccgga 1260
cagggaaggt gacaagaaag gtgtgtactg gtacaacaat aagttatgag gatgatggtg 1320
aagaaattgt cgacctttct cttgtctgtt tgtcttttgt taaagaagct atgcttcgtt 1380
ttaataatct tattgtccat tttgttgtgt tatgacattt tggctgctca ttatgttatg 1440
tgggaagtta gtgttcaaat gttttgtgtc ggtattgttc ttctcatcgc tgttttgttg 1500
ggatcgtaga aatgtgacct tcggacagta aaactcttgt actaaaacta tctccctatt 1560
ggcatttctt aaacttttaa tagttacgtg cctcgtagtg aatcttgact tgagcca 1617

<210> 18
<211> 1330
<212> DNA
<213> Arabidopsis thaliana

<220>
<221> misc_feature
<223> TC160629 (PRO0194)

<400> 18
ctcactcact attttcacat tctcttctct ctcgatatca tctaaatctc tctcgatctc 60
aatttcgcaa aatggctgac aagaagatca gaatcggaat caacggtttc ggaagaatcg 120
gtcgtttggt tgctagagtt gttcttcaga gggatgatgt tgagctcgtc gctgttaacg 180
atcctttcat caccaccgag tacatgacat acatgtttaa gtatgacagt gttcacggtc 240
agtggaagca ccatgagctt aaggtgaagg atgacaaaac tcttctcttc ggtgagaagc 300
cagtcactgt tttcggcatc aggaaccctg aggacatccc atggggtgag gctggagctg 360
actttgttgt tgagtctact ggtgtcttca ctgacaaaga caaggctgct gctcacttga 420
agggtggtgc taaaaaggtt gtcatctctg ccccaagcaa agatgcgccc atgttcgttg 480
ttggtgtcaa cgagcacgag tacaagtctg accttgacat tgtttccaac gctagttgca 540
ccactaactg ccttgctcct cttgccaagg ttattaatga caggtttggc attgttgagg 600
gactcatgac cactgtccac tctatcactg ctactcagaa gacagttgat ggtccatcaa 660
tgaaggactg gagaggtgga agagctgctt ccttcaacat tattcctagc agcactggtg 720
ccgccaaggc tgttgggaaa gtgttgccat ccctcaatgg aaaattgacc ggaatgtctt 780
tccgtgttcc aaccgttgat gtctcagttg ttgatctcac cgttagactt gagaaagctg 840
caacatacga cgaaatcaag aaggccatca aggaggaatc tgaaggcaaa atgaagggaa 900
ttttgggata cactgaggat gatgttgtgt ctaccgactt tgttggtgac aacaggtcaa 960
gcattttcga tgccaaggct gggattgcat tgagcgacaa gtttgtgaag ttggtgtcat 1020
ggtacgacaa cgaatggggt tacagttctc gtgtcgttga ccttatcgtt cacatgtcaa 1080
aggcctaagc ttacaccggc gagagtttgt gtgtggttga gttcgtactg ttctgaataa 1140
aaaaaaggag aaaagaaaac tcgagttgtt atgttttttc actgattcca tgcgcagtca 1200
tgagagtttg tagcttttgt ctttttgctt tctcttaatg tttccctgct ttatttactg 1260

aaaccattgg tttggttttt tatgttaatt aagtttttag ttcattactt tctcgtgcta   1320
actcaaaccc                                                          1330

<210>  19
<211>  58
<212>  DNA
<213>  Artificial sequence

<220>
<223>  primer PRM3275

<400>  19
ggggacaagt ttgtacaaaa aagcaggcta atactaatag aggaacagag tggtgttg       58

<210>  20
<211>  54
<212>  DNA
<213>  Artificial sequence

<220>
<223>  primer PRM2943

<400>  20
ggggacaagt ttgtacaaaa aagcaggctg gtgtttaaga atagaggaag gatg           54

<210>  21
<211>  54
<212>  DNA
<213>  Artificial sequence

<220>
<223>  primer PRM2945

<400>  21
ggggacaagt ttgtacaaaa aagcaggctc tcctcagcca tctgcctatc cacc           54

<210>  22
<211>  56
<212>  DNA
<213>  Artificial sequence

<220>
<223>  primer PRM3273

<400>  22
ggggacaagt ttgtacaaaa aagcaggcta ttgcttcgtc ggcagcgaat gaagcc         56

<210>  23
<211>  55
<212>  DNA
<213>  Artificial sequence

<220>
<223>  primer PRM4475

<400>  23
ggggacaagt ttgtacaaaa aagcaggctc acatgcactc tatagtttta tgtcg          55

<210>  24
<211>  56
<212>  DNA
<213>  Artificial sequence

<220>
<223>  primer PRM4479

<400>  24
ggggacaagt ttgtacaaaa aagcaggctc agattcagca cacaaagcca gtaaag         56

<210>  25
<211>  53
<212>  DNA
<213>  Artificial sequence

<220>
<223>  primer PRM4489

<400>  25
ggggacaagt ttgtacaaaa aagcaggctt ccagatacat catgggcttt ggg            53

<210>  26
<211>  56
<212>  DNA
<213>  Artificial sequence

<220>
<223>  primer PRM4498

<400>  26
ggggacaagt ttgtacaaaa aagcaggcta aacagtggtt aggattgtat gacgcc         56

<210>  27
<211>  56
<212>  DNA
<213>  Artificial sequence

<220>
<223>  primer PRM4500

<400>  27
ggggacaagt ttgtacaaaa aagcaggctg aggaggagaa gaagaagact tttggc         56

<210>  28
<211>  56
<212>  DNA
<213>  Artificial sequence

<220>

<223>  primer PRM3276

<400>  28
ggggaccact ttgtacaaga aagctgggta gttggacgga gagatcgaga aatcag          56

<210>  29
<211>  54
<212>  DNA
<213>  Artificial sequence

<220>
<223>  primer PRM2944

<400>  29
ggggaccact ttgtacaaga aagctgggtg gggtttggtt tgattgttgt gttg            54

<210>  30
<211>  55
<212>  DNA
<213>  Artificial sequence

<220>
<223>  primer PRM2946

<400>  30
ggggaccact ttgtacaaga aagctgggtt gtgggggact aaactcaaga atgag           55

<210>  31
<211>  54
<212>  DNA
<213>  Artificial sequence

<220>
<223>  primer PRM3274

<400>  31
ggggaccact ttgtacaaga aagctgggta tctcaagact gcgacgagta tgcg            54

<210>  32
<211>  57
<212>  DNA
<213>  Artificial sequence

<220>
<223>  primer PRM4476

<400>  32
ggggaccact ttgtacaaga aagctgggtt cttcaccgga taatgttcag tcttagc         57

<210>  33
<211>  56
<212>  DNA
<213>  Artificial sequence

<220>
<223>  primer PRM4480

<400>  33
ggggaccact ttgtacaaga aagctgggtt ttctttttgt ttgttgtgag agatgg        56

<210>  34
<211>  60
<212>  DNA
<213>  Artificial sequence

<220>
<223>  primer PRM4490

<400>  34
ggggaccact ttgtacaaga aagctgggtg cttttccttt cgtctctcta ataatttatg    60

<210>  35
<211>  60
<212>  DNA
<213>  Artificial sequence

<220>
<223>  primer PRM4499

<400>  35
ggggaccact ttgtacaaga aagctgggtg tttctgcaga aaaccaaaag caaaagaatc    60

<210>  36
<211>  59
<212>  DNA
<213>  Artificial sequence

<220>
<223>  primer PRM4501

<400>  36
ggggaccact ttgtacaaga aagctgggtt ttgcgaaatt gagatcgaga gagatttag     59

## Description

The present invention relates to the field of plant molecular biology, more particularly to nucleic acid sequences useful for driving and/or regulating expression of an operably linked nucleic acid in a plant. The isolation of these nucleic acid sequences from *Arabidopsis thaliana,* as well as their use in driving and/or regulating expression of an operably linked nucleic acid is disclosed. The present invention therefore concerns promoters, hybrid promoters, genetic constructs, expression cassettes, transformation vectors, expression vectors, host cells and transgenic plants comprising a promoter according to the present invention. The present invention also concerns methods for driving and/or regulating expression of a nucleic acid and methods for the production of transgenic plants comprising a promoter according to the present invention.

Gene expression is dependent on initiation of transcription, which is mediated via a transcription initiation complex. Regulation of transcription to determine how strong, when or where a gene is expressed may be mediated via transcriptional control elements, which are generally embedded in the nucleic acid sequence 5'-flanking or upstream of the expressed gene. This upstream nucleic acid region is often referred to as a "promoter" since it promotes the binding, formation and/or activation of the transcription initiation complex and is therefore capable of driving and/or regulating expression of the 3' downstream nucleic acid sequence.

Genetic engineering of plants, aimed at obtaining useful plant phenotypes, often involves heterologous gene expression, which is generally mediated by a promoter capable of driving and/or regulating expression of an operably linked heterologous nucleic acid. The phenotype of the host plant depends not only on the contribution of the heterologous nucleic acid, but also on the contribution of the specific expression pattern of the chosen promoter determining how, where and when that heterologous nucleic acid is expressed. Accordingly, the choice of promoter with a suitable expression pattern is of crucial importance for obtaining the desired plant phenotype. A person skilled in the art will need to have available different promoters, to determine the optimal promoter for a particular (heterologous) nucleic acid. This availability is rather limited and there is therefore a continuing need to provide new promoters with various expression profiles in plants.

The nucleic acids as represented by SEQ ID NO 1 to 9 were isolated from *Arabidopsis thaliana* and have been found to be capable of driving and regulating expression of an operably linked (heterologous) nucleic acid. Therefore the present invention offers a collection of nucleic acids which have been isolated for the fist time and these nucleic acids have been found to act as promoters and their expression patterns are now disclosed for the first time. It is demonstrated that these isolated nucleic acids are useful as promoters in heterologous gene expression.

Accordingly, the present invention provides an isolated promoter capable of driving and/or regulating expression, comprising:
(a) an isolated nucleic acid as represented by any one of SEQ ID NO 1 to 9 or the complement of any one of SEQ ID NO 1 to 9; or
(b) an isolated nucleic acid having at least 90% sequence identity with any one of the DNA sequences as represented by any one of SEQ ID NO 1 to 9; or
(c) an isolated nucleic acid specifically hybridizing under stringent conditions with any one of the DNA sequences as represented by any one of SEQ ID NO 1 to 9; or
(d) an isolated nucleic acid as defined in any one of (a) to (c), which is interrupted by an intervening sequence; or
(e) a fragment of any one of the nucleic acids as defined in (a) to (d), which fragment is capable of driving and/or regulating expression.

The term "isolated" as used herein means being removed from its original source. Preferably, the isolated promoter is free of sequences (such as protein-encoding sequences or other sequences at the 3' end) that naturally flank the promoter in the genomic DNA of the organism from which the promoter is derived. Further preferably, the isolated promoter is also free of sequences that naturally flank it at the 5' end. Further preferably, the isolated promoter may comprise less than about 5 kb, 4 kb, 3 kb, 2 kb, 1.5 kb, 1.2 kb, 1 kb, 0.8 kb, 0.5 kb or 0.1 kb of nucleotide sequences that naturally occur with the promoter in genomic DNA from the organism of which the promoter is derived.

The present invention is not limited to the nucleic acids as represented by SEQ ID NO 1 to 9. A person skilled in the art will recognize that variants or fragments of a nucleic acid may occur, whilst maintaining the same functionality. These variants or fragments may occur in nature or may be man made (e.g. by genetic engineering). Therefore the present invention extends to variant nucleic acids and fragments of any one of SEQ ID NO 1 to 9, which variants or fragments are useful in the methods of the present invention. Such variants and fragments include:
(a) an isolated nucleic acid as represented by any one of SEQ ID NO 1 to 9 or the complement of any one of SEQ ID NO 1 to 9; or
(b) an isolated nucleic acid having at least 90% sequence identity with any one of the DNA sequences as represented by any one of SEQ ID NO 1 to 9; or
(c) an isolated nucleic acid specifically hybridizing under stringent conditions with any one of the DNA sequences as represented by any one of SEQ ID NO 1 to 9; or
(d) an isolated nucleic acid as defined in any one of (a) to (c), which is interrupted by an intervening sequence; or
(e) a fragment of any one of the nucleic acids as defined in (a) to (d), which fragment is capable of driving and/or regulating expression.

Suitable variants of any one of SEQ ID NO 1 to 9 encompass homologues which have in increasing order of preference at least about 90%, 91%, 92%, 93%, 94%, 95%, 96%, 97%, 98% or 99% sequence identity with any one of the nucleic acids as represented by SEQ ID NO 1 to 9.

The percentage of sequence identity is calculated using a pairwise global alignment program implementing the algorithm of Needleman-Wunsch (J. Mol. Biol. 48: 443-453, 1970), which maximizes the number of matches and minimizes the number of gaps. For calculation of the above-mentioned percentages, the program Align X (as part of the Vector NTI suite 5.5) may be used with the standard parameters and the variable parameters gap opening penalty 10 and gap extension penalty 0.1. "Sequence identity" as used herein is preferably calculated over the entire length of the nucleic acid as represented by any one of SEQ ID NO 1 to 9. The length of these nucleic aicds is presented in Table 2.

Search and identification of homologous nucleic acids, would be well within the realm of a person skilled in the art. Such methods, involve screening sequence databases with the sequences provided by the present invention, for example any one of SEQ ID NO 1 to 9, preferably in a computer readable form. Useful sequence databases, include, but are not limited to, Genbank (http://www.ncbi.nlm.nih.gov/web/Genbank), the European Molecular Biology Laboratory Nucleic acid Database (EMBL) (http:/w.ebi.ac.uk/ebi-docs/embl-db.html) or versions thereof, or the MIPS database (http://mips.gsf.de/). Different search algorithms and software for the alignment and comparison of sequences are well known in the art. Such software includes, for example GAP, BESTFIT, BLAST, FASTA and TFASTA. Preferably BLAST software is used, which calculates percent sequence identity and performs a statistical analysis of the similarity between the sequences. The suite of programs referred to as BLAST programs has 5 different implementations: three designed for nucleotide sequence queries (BLASTN, BLASTX, and TBLASTX) and two designed for protein sequence queries (BLASTP and TBLASTN) (Coulson, Trends in Biotechnology: 76-80, 1994; Birren et al., GenomeAnalysis, 1: 543, 1997). The software for performing BLAST analysis is publicly available through the National Centre for Biotechnology Information.

The sequences of the genome of *Arabidopsis thaliana* and the genome of *Oryza sativa* are now available in public databases such as Genbank. Other genomes are currently being sequenced. Therefore, it is expected that as more sequences of the genomes of other plants become available, homologous promoters may be identifiable by sequence alignment with any one of SEQ ID NO 1 to SEQ ID NO 9. The skilled person will readily be able to find homologous promoters from other plant species, for example from other dicotyledonous plants, such as other members of the *Brassicaceae* family or from other plant families. Homologous promoters from crop plants are especially useful for practising the methods of the present invention in crop plants.

One example of homologues having at least 90% sequence identity with any one of SEQ ID NO 1 to 9 are allelic variants of any one of SEQ ID NO 1 to 9. Allelic variants are variants of the same gene occurring in two different individuals of the same species and usually allelic variants differ by slight sequence changes. Allelic variants may encompass Single Nucleotide Polymorphisms (SNPs) as well as Small Insertion/Deletion Polymorphisms (INDELs). The size of INDELs is usually less than 100 bp. SNPs and INDELs form the largest set of sequence variants in naturally occurring polymorphic strains of most organisms.

Homologues suitable for use in the methods according to the invention may readily be isolated from their source organism via the technique of PCR or hybridization. Their capability of driving and/or regulating expression may readily be determined, for example, by following the methods described in the Examples section by simply substituting the sequence used in the actual Example with the homologue.

Another variant of any one of SEQ ID NO 1 to 9 encompassed by the present invention is a nucleic acid specifically hybridising under stringent conditions to any one of the nucleic acids of SEQ ID NO 1 to 9. The term "hybridising" means annealing to substantially homologous complementary nucleotide sequences in a hybridization process. Tools in molecular biology relying on such a hybridization process include the polymerase chain reaction (PCR; and all methods based thereon), subtractive hybridisation, random primer extension, nuclease S1 mapping, primer extension, reverse transcription, cDNA synthesis, differential display of RNAs, and DNA sequence determination, Northern blotting (RNA blotting), Southern blotting (DNA blotting). The hybridisation process can also occur with one of the complementary nucleic acids immobilised to a matrix such as magnetic beads, Sepharose beads or any other resin. Tools in molecular biology relying on such a process include the isolation of poly (A+) mRNA. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). Tools in molecular biology relying on such a process include RNA and DNA gel blot analysis, colony hybridisation, plaque hybridisation, *in situ* hybridisation and microarray hybridisation. In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration and hybridisation buffer composition. Conventional hybridisation conditions are described in, for example, Sambrook (2001) Molecular Cloning: a laboratory manual, 3rd Edition Cold Spring Harbor Laboratory Press, CSH, New York, but the skilled craftsman will appreciate that numerous different hybridisation conditions may be designed in function of the known or the expected homology and/or length of the nucleic acid sequence. High stringency conditions for hybridisation include high temperature and/or low sodium/salt concentration (salts include sodium as for example in NaCl and Na₃-citrate) and/or the inclusion of formamide in the hybridisation buffer and/or lowering the concentration of compounds such as SDS (sodium dodecyl sulphate detergent) in the hybridisation buffer and/or exclusion of compounds such as dextran sulphate or polyethylene glycol (promoting molecular crowding) from the hybridisation buffer. Specific hybrisization under stringent conditions is preferably carried out at a temperature of 60°C followed by washes in 0.1 to 1 XSSC, 0.1XSDS, and 1X SSC, 0.1X SDS. Sequences capable of specifically hybridising under stringent conditions are sequences that are very similar.

The invention also relates to a nucleic acid molecule of at least 15 nucleotides in length hybridizing specifically with any one of the nucleic acids of the invention. The invention also relates to a nucleic acid molecule of at least 15 nucleotides in length specifically amplifying a nucleic acid of the invention by polymerase chain reaction.

Another variant of any one of SEQ ID NO 1 to 9 encompassed by the present invention is a variant, which is interrupted by an intervening sequence. For example, any one of the nucleic acids as represented by SEQ ID NO 1 to 9 may be interrupted by an intervening sequence. With "intervening sequence" is meant any nucleic acid or nucleotide, which disrupts another sequence. Examples of intervening sequences include introns, nucleic acid tags, T-DNA and mobilizable nucleic acids sequences such as transposons or nucleic acids that may be mobilized via recombination. Examples of particular transposons comprise Ac (activator), Ds (Dissociation), Spm (suppressor-Mutator) *or En.* The introduction of introns into promoters is now widely applied. The methods according to the present invention may also be practised using a nucleic acid sequence according to any one of SEQ ID NO 1 to 9 provided with an intron. In case the intervening sequence is an intron, alternative splice variants of the nucleic acids according to the invention may arise. The term "alternative splice variant" as used herein encompasses variants of a nucleic acid sequence in which intervening introns have been excised, replaced or added. Such splice variants may be found in nature or may be manmade. Methods for making such promoters with an intron or for making the corresponding splice variants are well known in the art.

Variants interrupted by an intervening sequence, suitable for use in the methods according to the invention may readily be determined for example by following the methods described in the Examples section by simply substituting the sequence used in the actual Example with the variant.

The variant nucleic acids as described hereinabove may be found in nature (for example allelic variants or splice variants). Additionally and/or alternatively, variants of any one of SEQ ID NO 1 to 9 as described hereinabove may be manmade via techniques well known in the art involving for example mutation, substitution, insertion, deletions or derivation. The present invention also encompasses such variants, as well as their use in the methods of the present invention.

A "mutation variant" of a nucleic acid may readily be made using recombinant DNA manipulation techniques or nucleotide synthesis. Examples of such techniques include site directed mutagenesis via M13 mutagenesis, T7-Gen *in vitro* mutagenesis (USB, Cleveland, OH), QuickChange Site Directed mutagenesis (Stratagene, San Diego, CA), PCR-mediated site-directed mutagenesis or other site-directed mutagenesis protocols. Alternatively, the nucleic acid of the present invention may be randomly mutated, for example by "error prone PCR".

A "substitutional variant" refers to those variants in which at least one residue in the nucleic acid sequence has been removed and a different residue inserted in its place. Nucleic acid substitutions are typically of single residues, but may be clustered depending upon functional constraints placed upon the nucleic acid sequence; insertions usually are of the order of about 1 to about 10 nucleic acid residues, and deletions can range from about 1 to about 20 residues.

An "insertional variant" of a nucleic acid is a variant in which one or more nucleic acid residues are introduced into a predetermined site in that nucleic acid. Insertions may comprise 5'-terminal and/or 3'-terminal fusions as well as intra-sequence insertions of single or multiple nucleotides. Generally, insertions within the nucleic acid sequence will be smaller than 5'- or 3'-terminal fusions, of the order of about 1 to 10 residues. Examples of 5'- or 3'-terminal fusions include the coding sequences of binding domains or activation domains of a transcriptional activator as used in the yeast two-hybrid system or yeast one-hybrid system, or of phage coat proteins, (histidine)₆-tag, glutathione S-transferase-tag, protein A, maltose-binding protein, dihydrofolate reductase, Tag•100 epitope, c-myc epitope, FLAG^{®}-epitope, lacZ, CMP (calmodulin-binding peptide), HA epitope, protein C epitope and VSV epitope.

The term "derivative" of a nucleic acid may comprise substitutions, and/or deletions and/or additions of naturally and non-naturally occurring nucleic acid residues compared to the natural nucleic acid. Derivatives may, for example, comprise methylated nucleotides, or artificial nucleotides.

Also encompassed by the present invention are fragments of the nucleic acids as represented by any one of SEQ ID NO 1 to 9 or variants thereof as described hereinabove. A "fragment" as used herein means a portion of a nucleic acid sequence. Suitable fragments useful in the methods of the present invention are functional fragments, which retain at least one of the functional parts of the promoter and hence are still capable of driving and/or regulating expression. Examples of functional fragments of a promoter include the minimal promoter, the upstream regulatory elements, or any combination thereof.

Suitable fragments may range from at least about 20 base pairs to about 50, 100, 150, 200, 250, 300, 350, 400, 450, 500, 550, 600, 650, 700, 750, 800, 850, 900, 950 or 1000 base pairs, up to about the full length sequence of the invention. These base pairs are typically immediately upstream of the transcription initiation start, but alternatively may be from anywhere in the promoter sequence.

Suitable means to find functional fragments (i.e. fragments comprising regulatory regions, regulatory elements, cis-elements, boxes or minimal promoter) include software programs designed for motif searching. Preferred computer programs include MEME, SIGNALSCAN, and GENESCAN. A MEME algorithm (Version 2.2) is found in version 10.0 of the GCG package; or on the internet site http://www.sdsc.edu/MEME/meme. SIGNALSCAN version 4.0 information is available on the internet site http://biosci.cbs.umn.edu/software/sigscan.html. GENESCAN may be found on the internet site http://gnomic.stanford.edu/GENESCANW.html.

Suitable fragments useful in the methods of the present invention may be tested for their capability of driving and/or regulating expression by standard techniques well known to the skilled person, or by the following method described in the Example section.

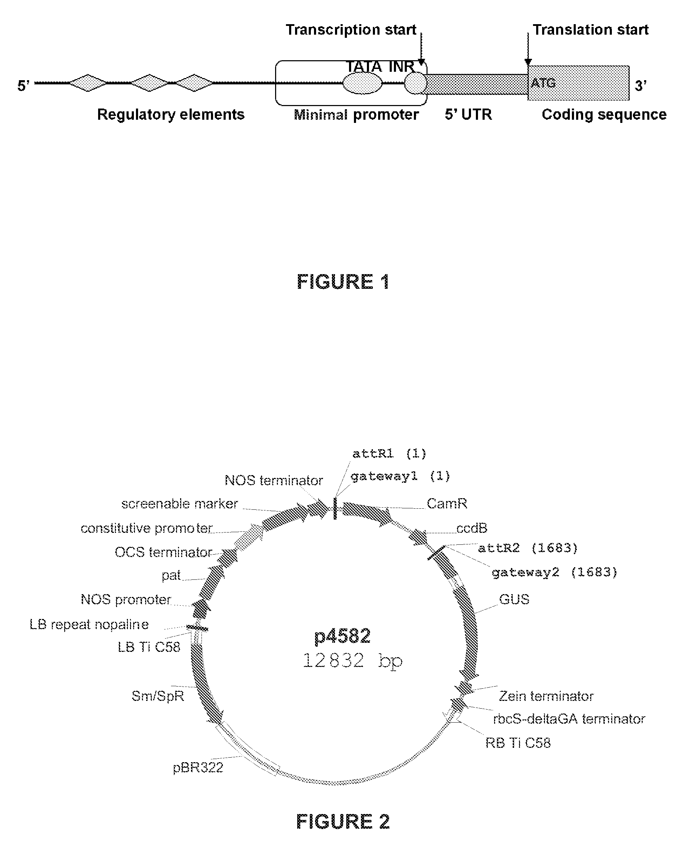

The promoters as disclosed in any one of SEQ ID NO 1 to 9 are isolated as nucleic acids of approximately 1.2kb from the upstream region of particular *Arabidopsis* coding sequences (CDS). These nucleic acids may include typical elements of a promoter, which are presented in Figure 1. Generally, a promoter may comprises from coding sequence into the upstream direction: (i) a 5'UTR of pre-messenger RNA, (ii) a minimal promoter comprising the transcription initiation element (INR) and, more upstream, a TATA box, and (iii) may contain regulatory elements that determine the specific expression pattern of the promoter.

The term "promoter" as used herein is taken in a broad context and refers to regulatory nucleic acid sequences capable of effecting (driving and/or regulating) expression of the sequences to which they are operably linked. A "promoter" encompasses transcriptional regulatory sequences derived from a classical genomic gene. Usually a promoter comprises a TATA box, which is capable of directing the transcription initiation complex to the appropriate transcription initiation start site. However, some promoters do not have a TATA box (TATA-less promoters), but are still fully functional for driving and/or regulating expression. A promoter may additionally comprise a CCAAT box sequence and additional regulatory elements (i.e. upstream activating sequences or cis-elements such as enhancers and silencers). A "promoter" may also include the transcriptional regulatory sequences of a classical prokaryotic gene, in which case it may include a -35 box sequence and/or a -10 box transcriptional regulatory sequences.

"Driving expression" as used herein means promoting the transcription of a nucleic acid.

"Regulating expression" as used herein means influencing the level, time or place of transcription of a nucleic acid. The promoters of the present invention may thus be used to increase, decrease or change in time and/or place transcription of a nucleic acid. For example, they may be used to limit the transcription to certain cell types, tissues or organs, or during a certain period of time, or in response to certain environmental conditions.

The promoter is preferably a plant-expressible promoter. The term "plant-expressible" means being capable of regulating expression in a plant, plant cell, plant tissue and/or plant organ. Accordingly, the invention encompasses an isolated nucleic acid as mentioned above, capable of regulating transcription of an operably linked nucleic acid in a plant or in one or more particular cells, tissues or organs of a plant.

The expression pattern of the promoters according to the present invention was studied in detail and it was found that many of them were tissue-specific. Accordingly, the present invention provides "tissue-specific" promoters. The term "tissue-specific" shall be taken to indicate that expression is predominantly in a particular tissue, tissue-type, organ or any other part of the organism, albeit not necessarily exclusively in said tissue, tissue-type, organ or other part. Accordingly, the invention encompasses an isolated nucleic acid as mentioned above, capable of driving and/or regulating expression (of an operably linked nucleic acid) in a tissue-specific manner. Expression may be driven and/or regulated for example in the root, root meristem, root tip, lateral roots, root central cylinder, or in hypocotyls, cotyledons, meristem, shoot, shoot meristem, leaves, trichomes, hydathodes, apical meristem, flowers, petals, pedicle, stamen, siliques, seed, embryo.

A tissue-specific promoter is one example of a so-called "regulated promoter". These promoters are regulated by endogenous signals such as the presence of certain transcription factors, metabolites, plant hormones, or exogenous signals, such as ageing, stresses or nutritional status. These regulations may have an effect on one or more different levels such spatial specificity or temporal specificity. Encompassed within the present invention is a nucleic acid as described hereinabove, which is a "regulated promoter". Examples of regulated promoters are cell-specific promoters, tissue-specific promoters, organ-specific promoters, inducible promoters or young tissue-specific promoters.

Alternatively and/or additionally, some promoters of the present invention display a constitutive expression pattern. Accordingly, the present invention provides a promoter as described hereinabove, which is a constitutive promoter. The term "constitutive" means having no or very few spatial or temporal regulation. The term "constitutive expression" as used herein refers to substantially continuously expression in substantially all tissues of the organism. The skilled craftsman will understand that a "constitutive promoter" is a promoter that is active during most, but not necessarily all, phases of growth and development of the organism and throughout most, but not necessarily all, parts of an organism.

The "expression pattern" of a promoter is not only influenced by the spatial and temporal aspects, but also by the level of expression. The level of expression is determined by the so-called "strength" of a promoter. Depending on the resulting expression level, a distinction is made herein between "weak" and "strong" promoters. Generally by "weak promoter" is meant a promoter that drives expression of an operably linked nucleic acid at levels of about 1/10000 transcripts, to about 1/100000 transcripts or to about 1/500000 transcripts. Generally, by "strong promoter" is meant a promoter that drives expression at levels of about 1/10 transcripts, to about 1/100 or to about 1/1000 transcripts.

According to a particular embodiment, the invention provides an isolated promoter as mentioned hereinabove, which is a hybrid promoter. The term "hybrid promoter" as used herein refers to a chimeric promoter made, for example, synthetically, for example by genetic engineering. Preferred hybrid promoters according to the present invention comprise a part, preferably a functional part, of one of the promoters according to the present invention and at least one part of another promoter. Alternatively, hybrid promoters encompassed by the present invention comprise another part of the same promoter. One example of a hybrid promoter comprises regulatory element(s) of a promoter according to the present invention combined with the minimal promoter of another promoter. Another example of a hybrid promoter is a promoter comprising additional regulatory elements to further enhance its activity and/or to alter its spatial and/or temporal expression pattern.

The present invention also provides use of a functional fragment of any one of SEQ ID NO 1 to 9 or variant thereof for changing the expression pattern of a promoter. In such methods, at least part of any of the nucleic acids according to the present invention are combined with at least one fragment of another promoter. According to a particular embodiment the invention encompasses a method for conferring tissue-specificity, and/or constitutive expression to a promoter sequence, comprising the fusion of a promoter according to the present invention or at least a functional fragment thereof, to that promoter sequence normally not exhibiting that tissue specificity and/or constitutive expression. Such modifications and fusions may be achieved by routine experimentation by those skilled in the art.

Further, the invention provides a genetic construct comprising:
(a) an isolated promoter as defined hereinabove; and
(b) a heterologous nucleic acid sequence operably linked to the isolated promoter of (a); and optionally
(c) a 3' transcription terminator.

The term "genetic construct" as used herein means a nucleic acid made by genetic engineering.

The term "operably linked" to a promoter as used herein means that the transcription is driven and/or regulated by that promoter. A person skilled in the art will understand that being operably linked to a promoter preferably means that the promoter is positioned upstream (i.e. at the 5'-end) of the operably linked nucleic acid. The distance to the operably linked nucleic acid may be variable, as long as the promoter of the present invention is capable of driving and/or regulating the transcription of the operably linked nucleic acid. For example, between the promoter and the operably linked nucleic acid, there might be a cloning site, an adaptor, a transcription or translation enhancer.

The operably linked nucleic acid may be any coding or non-coding nucleic acid. The operably linked nucleic acid may be in a sense or in anti-sense direction. Typically in the case of genetic engineering of host cells, the operably linked nucleic acid is to be introduced into the host cell and is intended to change the phenotype of the host cell. Alternatively, the operably linked nucleic acid is an endogenous nucleic acid from the host cell.

The term "heterologous" as used herein is intended to be "heterologous to the promoter of the present invention". A nucleic acid that is heterologous to the promoter of the present invention is one that is not the naturally occurring nucleic acid sequence flanking the promoter of the present invention when it is in its biological genomic environment. While the nucleic acid may be heterologous to the promoter of the present invention, it may be homologous or native or heterologous or foreign to the plant host cell. The heterologous operably linked nucleic acid may be any nucleic acid (for example encoding any protein), provided that it comprises or it is flanked by at least one nucleotide which is normally not flanking the promoter of the present invention.

The term "transcription terminator" as used in (c) refers to a DNA sequence at the end of a transcriptional unit which signals termination of transcription. Terminators are 3'-non-translated DNA sequences usually containing a polyadenylation signal, which facilitates the addition of polyadenylate sequences to the 3'-end of a primary transcript. Terminators active in and/or isolated from viruses, yeasts, moulds, bacteria, insects, birds, mammals and plants are known and have been described in literature. Examples of terminators suitable for use in the genetic constructs of the present invention include the *Agrobacterium tumefaciens* nopaline synthase (NOS) gene terminator, the *Agrobacterium tumefaciens* octopine synthase (OCS) gene terminator sequence, the Cauliflower mosaic virus (CaMV) 35S gene terminator sequence, the *Oryza sativa* ADP-glucose pyrophosphorylase terminator sequence (t3'Bt2), the Zea *mays* zein gene terminator sequence, the *rbcs-1A* gene terminator, and the *rbcs-3A* gene terminator sequences, amongst others.

The present invention also provides an expression cassette, a transformation vector and a plant expression vector comprising a genetic construct as described above.

An "expression cassette" as meant herein refers to a minimal genetic construct necessary for expression of a nucleic acid. A typical expression cassette comprises a promoter-gene-terminator combination. An expression cassette may additionally comprise cloning sites, for example Gateway^{™} recombination sites or restriction enzyme recognition sites, to allow easy cloning of the operably linked nucleic acid to the promoter of the present invention or to allow the easy transfer of the expression cassette into a vector. An expression cassette may further comprise 5' untranslated regions, 3' untranslated regions, a selectable marker, transcription enhancers or translation enhancers.

With "transformation vector" is meant a genetic construct, which may be introduced in an organism by transformation and may be stably maintained in said organism. Some vectors may be maintained in, for example *Escherichia coli, A. tumefaciens, Saccharomyces cerevisiae* or *Schizosaccharomyces pombe,* while others such as phagemids and cosmid vectors, may be maintained in bacteria and/or viruses. Transformation vectors may be multiplied in their host cell and may be isolated again therefrom to be transformed into another host cell. Vector sequences generally comprise a set of unique sites recognized by restriction enzymes, the multiple cloning site (MCS), wherein one or more non-vector sequence(s) may be inserted. Vector sequences may further comprise an origin of replication which is required for maintenance and/or replication in a specific host cell. Examples of origins of replication include, but are not limited to, the f1-ori and colE1.

"Expression vectors" form a subset of transformation vectors, which, by virtue of comprising the appropriate regulatory sequences, enable expression of the inserted non-vector sequence(s). Expression vectors have been described which are suitable for expression in bacteria (e.g. *E*. *coli*)*,* fungi (e.g. *S. cerevisiae, S. pombe, Pichia pastoris),* insect cells (e.g. baculoviral expression vectors), animal cells (e.g. COS or CHO cells) and plant cells. One suitable expression vector according to the present invention is a plant expression vector, useful for the transformation of plant cells, the stable integration in the plant genome, the maintenance in the plant cell and the expression of the non-vector sequences in the plant cell.

Typically, a plant expression vector according to the present invention comprises a nucleic acid of any one of SEQ ID NO 1 to 9 or a variant thereof as described hereinabove, optionally operably linked to a second nucleic acid. Typically, a plant expressible vector according to the present invention, further comprises T-DNA regions for stable integration into the plant genome (for example the left border and the right border regions of the Ti plasmid).

The genetic constructs of the invention may further comprise a "selectable marker". As used herein, the term "selectable marker" includes any gene, which confers a phenotype to a cell in which it is expressed, to facilitate the identification and/or selection of cells that are transfected or transformed. Suitable markers may be selected from markers that confer antibiotic or herbicide resistance. Cells containing the genetic construct will thus survive antibiotics or herbicide concentrations that kill untransformed cells. Examples of selectable marker genes include genes conferring resistance to antibiotics (such as nptll encoding neomycin phosphotransferase capable of phosphorylating neomycin and kanamycin, or hpt encoding hygromycin phosphotransferase capable of phosphorylating hygromycin), to herbicides (for example bar which provides resistance to Basta; aroA or gox providing resistance against glyphosate), or genes that provide a metabolic trait (such as manA that allows plants to use mannose as sole carbon source). Visual marker genes result in the formation of colour (for example beta-glucuronidase, GUS), luminescence (such as luciferase) or fluorescence (Green Fluorescent Protein, GFP, and derivatives thereof). Further examples of suitable selectable marker genes include the ampicillin resistance (Ampr), tetracycline resistance gene (Tcr), bacterial kanamycin resistance gene (Kanr), phosphinothricin resistance gene, and the chloramphenicol acetyltransferase (CAT) gene, amongst others.

Furthermore, the present invention encompasses a host cell comprising an isolated promoter, or a genetic construct, or an expression cassette, or a transformation vector or an expression vector according to the invention as described hereinabove. In particular embodiments of the invention, the host cell is selected from bacteria, algae, fungi, yeast, plants, insect or animal host cells.

In one particular embodiment, the invention provides a transgenic plant cell comprising an isolated promoter according to the invention, or an isolated nucleic acid, or a genetic construct, or an expression cassette, or a transformation vector or an expression vector according to the invention as described hereinabove. Preferably said plant cell is a dicot plant cell or a monocot plant cell, more preferably a cell of any of the plants as mentioned herein. Preferably, in the transgenic plant cell according to the invention, the promoter or the genetic construct of the invention is stably integrated into the genome of the plant cell.

The invention also provides a method for the production of a transgenic plant, comprising:
(a) introducing into a plant cell an isolated promoter according to the invention, for example as represented by any one of SEQ ID NO 1 to SEQ ID NO 9, or a variant or fragment thereof, or a genetic construct, or an expression cassette, or a transformation vector or an expression vector according to the present invention and as described hereinabove, and
(b) cultivating said plant cell under conditions promoting plant growth.

"Introducing" the above mentioned isolated promoter, or genetic construct, or expression cassette, or transformation vector or expression vector, into a host cell (e.g. plant cell) is preferably achieved by transformation. The term "transformation" as used herein encompasses the transfer of an exogenous polynucleotide into a host cell, irrespective of the method used for transfer. In particular for plants, tissues capable of clonal propagation, whether by organogenesis or embryogenesis, are suitable for transformation with a genetic construct of the present invention and a whole plant may be regenerated therefrom. The particular tissue chosen will vary depending on the clonal propagation systems available for, and best suited to, the particular plant species being transformed. Exemplary tissue targets include leaf disks, pollen, embryos, cotyledons, hypocotyls, megagametophytes, callus tissue, existing meristematic tissue (e.g. apical meristem, axillary buds, and root meristems), and induced meristem tissue (e.g. cotyledon meristem and hypocotyl meristem). The polynucleotide may be transiently or stably introduced into a plant cell and may be maintained non-integrated, for example as a plasmid. Alternatively, it may be integrated into the plant genome.

Transformation of a plant species is now a fairly routine technique. Advantageously, any of several transformation methods may be used to introduce the nucleic acids of the invention into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et a/., 1882, Nature 296, 72-74; Negrutiu 1. et al., June 1987, Plant Mol. Biol. 8, 363-373); electroporation of protoplasts (Shillito R.D. et a/., 1985 Bio/Technol 3, 1099-1102); microinjection into plant material (Crossway A. et a/., 1986, Mol. Gen Genet 202, 179-185); DNA or RNA-coated particle bombardment (Klein T.M. et al., 1987, Nature 327, 70) infection with (non-integrative) viruses and the like. A preferred transformation method for the production of transgenic plant cells according to the present invention is an *Agrobacterium* mediated transformation method.

Transgenic rice plants comprising any one of the promoters of the present invention are preferably produced via Agrobacterium-mediated transformation using any of the well-known methods for rice transformation, such as the ones described in any of the following: published European patent application EP 1198985 A1, Aldemita and Hodges (Planta, 199, 612-617, 1996); Chan et al. (Plant Mol. Biol. 22 (3) 491-506, 1993); Hiei et al. (Plant J. 6 (2) 271-282, 1994); which disclosures are incorporated by reference herein as if fully set forth. In the case of corn transformation, the preferred method is as described in either Ishida et al. (Nat. Biotechnol. 1996 Jun; 14(6): 745-50) or Frame et al. (Plant Physiol. 2002 May; 129(1): 13-22), which disclosures are incorporated by reference herein as if fully set forth.

Generally after transformation, plant cells or cell groupings are selected for the presence of one or more markers which are encoded by plant-expressible genes co-transferred with the nucleic acid of interest, following which the transformed material may be cultivated under conditions promoting plant growth.

The resulting transformed plant cell may then be used to regenerate a transformed plant in a manner known to persons skilled in the art. Accordingly, the method for the production of a transgenic plant as described hereinabove, may further comprise regenerating a plant from said plant cell of (a).

The present invention further provides a plant comprising a plant cell as described hereinabove. The plants may also be able to grow, or even reach maturity including for example fruit production, seed formation, seed ripening and seed setting.

Furthermore, progeny may be produced from these seeds, which progeny may be fertile. Alternatively or additionally, the transformed and regenerated plants may also produce progeny by non-sexual propagation such as cloning, grafting. The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

Following DNA transfer and growth of the transformed cells, putatively transformed plant cells or plants may be evaluated, for instance using Southern analysis, for the presence of the gene of interest, copy number and/or genomic organization. Alternatively or additionally, expression levels or expression patterns of the newly introduced DNA may be undertaken using northern and/or Western analysis, both techniques being well known to persons having ordinary skill in the art.

The present invention clearly extends to plants obtainable by any of the methods according to the present invention, which plants comprise any of the isolated promoters or the constructs of the present invention. The present invention clearly extends to any plant parts and propagules of such plant. The present invention extends further to encompass the progeny of a primary transformed cell, tissue, organ or whole plant that has been produced by any of the aforementioned methods, the only requirement being that progeny exhibit the same genotypic and/or phenotypic characteristic(s) as those produced in the parent by the methods according to the invention. The invention also extends to harvestable parts of a plant, such as but not limited to seeds, leaves, fruits, flowers, stem cultures, stem, rhizomes, roots, tubers, bulbs and cotton fibers.

The term "plant" or "plants" as used herein encompasses whole plants, ancestors and progeny of plants and plant parts, including seeds, shoots, stems, roots (including tubers), and plant cells, tissues and organs. The term "plant" therefore also encompasses suspension cultures, embryos, meristematic regions, callus tissue, gametophytes, sporophytes, pollen, and microspores. Plants that are particularly useful in the methods of the invention include all plants which belong to the superfamily *Viridiplantae,* in particular monocotyledonous and dicotyledonous plants including a fodder or forage legume, ornamental plant, food crop, tree, or shrub selected from the list comprising *Acacia spp., Acer spp., Actinidia spp.,Aesculus spp., Agathis australis, Albizia amara, Alsophila tricolor, Andropogon spp., Arachis spp, Areca catechu, Astelia fragrans, Astragalus cicer, Baikiaea plurijuga, Betula spp., Brassica spp., Bruguiera gymnorrhiza, Burkea africana, Butea frondosa, Cadaba farinosa, Calliandra spp, Camellia sinensis, Canna indica, Capsicum spp., Cassia spp., Centroema pubescens, Chaenomeles spp., Cinnamomum cassia,* Coffea *arabica, Colophospermum mopane, Coronillia varia, Cotoneaster serotina, Crataegus spp., Cucumis spp., Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Diheteropogon amplectens, Dioclea spp, Dolichos spp., Dorycnium rectum, Echinochloa pyramidalis, Ehrartia spp., Eleusine coracana, Eragrestis spp., Erythrina spp., Eucalyptus spp., Euclea schimperi, Eulalia villosa, Fagopyrum spp., Feijoa sellowiana, Fragaria spp., Flemingia spp, Freycinetia banksii, Geranium thunbergii, Ginkgo biloba, Glycine javanica, Gliricidia spp, Gossypium hirsutum, Grevillea spp., Guibourtia coleosperma, Hedysarum spp., Hemarthia altissima, Heteropogon contortus, Hordeum vulgare, Hyparrhenia rufa, Hypericum erectum, Hyperthelia dissoluta, Indigo incarnata, Iris spp., Leptarrhena pyrolifolia, Lespediza spp., Lettuca spp., Leucaena leucocephala, Loudetia simplex, Lotonus bainesii, Lotus spp., Macrotyloma axillare, Malus spp., Manihot esculenta, Medicago sativa, Metasequoia glyptostroboides, Musa sapientum, Nicotianum spp., Onobrychis spp., Ornithopus spp., Oryza spp., Peltophorum africanum, Pennisetum spp., Persea gratissima, Petunia spp., Phaseolus spp., Phoenix canariensis, Phormium cookianum, Photinia spp., Picea glauca, Pinus spp., Pisum sativum, Podocarpus totara, Pogonarthria fleckii, Pogonarthria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes grossularia, Ribes spp., Robinia pseudoacacia, Rosa spp., Rubus spp., Salix spp., Schyzachyrium sanguineum, Sciadopitys verticillata, Sequoia sempervirens, Sequoiadendron giganteum, Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp., Tsuga heterophylla, Vaccinium spp., Vicia spp. Vitis vinifera, Watsonia pyramidata, Zantedeschia aethiopica, Zea mays,* amaranth, artichoke, asparagus, broccoli, brussel sprout, cabbage, canola, carrot, cauliflower, celery, collard greens, flax, kale, lentil, oilseed rape, okra, onion, potato, rice, soybean, straw, sugarbeet, sugar cane, sunflower, tomato, squash, and tea, trees and algae amongst others. According to a preferred feature of the present invention, the plant is a crop plant such as soybean, sunflower, canola, alfalfa, rapeseed, cotton, tomato, potato, tobacco, squash, papaya, poplar, leguminosa, flax, lupinus or sorghum. According to another preferred embodiment of the present invention the plant is a monocotyledonous plant, such as sugarcane, further preferable a cereal such as rice, maize, wheat, barley, millet, rye or oats.

The invention further provides a method for driving and/or regulating expression of a nucleic acid in a plant or plant cell, comprising:
(a) operably linking a nucleic acid to a promoter according to the invention as described hereinabove, such as to any one of SEQ ID NO 1 to 9 or a variant thereof, and
(b) introducing the resultant genetic construct into a plant or plant cell.

Preferably the operably linked nucleic acid of (a) is heterologous to the nucleic acids according to the present invention.
Preferably the genetic construct is stably introduced into the plant or plant cell.

This method may further comprise cultivating the transformed plant or plant cell under conditions promoting growth, promoting regeneration and/or promoting maturation.

Furthermore, the expression of the operably linked nucleic acid may be driven and/or regulated in particular cells, tissues or organs of a plant. Accordingly, the invention provides a method as described above, wherein the expression is constitutive expression or tissue-specific expression. For these embodiments, reference is made to the example section where the specific expression patterns of the promoters according to the invention are described and where different types of tissue-specific expression are detailed.

The present invention further encompasses the use of an isolated promoter as defined hereinabove to drive and/or regulate expression of an operably linked nucleic acid.

(i) The person skilled in the art will recognize that provision of sequences SEQ ID NO 1 to 9, readily makes available the tools to isolate related promoters, which may have substantial sequence identity to any one of SEQ ID NO 1 to 9. Additionally, provision of sequences SEQ ID NO 10 to 18 (CDS corresponding to the promoters of the present invention, see Table 1), readily makes available the tools to isolate related promoters, of which the related CDSs may have substantial sequence identity to any one of SEQ ID NO 10 to 18. Therefore the present invention also encompasses a method for isolating nucleic acids, capable of driving and/or regulating expression of an (heterologous) operably linked nucleic acid, comprising screening a nucleic acid sequence database to find homologues of any of the sequences represented by SEQ ID NO 1 to 9 or SEQ ID NO 10 to 18. Subsequently synthetic nucleic acid that correspond to the sequence of these homologues are used to screen a library with genomic DNA, which library is for example prepared from the organism of origin of the above mentioned homologue. The screening procedure may for example involve hybridization. Subsequently, the genomic DNA that matches the homologue, is analysed to identify the transcription initiation site and the translation initiation site of the gene corresponding to the homologue. Finally, specific primers are designed for amplification of a nucleic acid located in the region upstream (at the 5' end) of said translation initiation site.

The present invention also extends to the identification of regulatory proteins that are involved in the regulation of the activity of the promoters according to the present invention. Such identification may be achieved using a yeast one-hybrid system. In such a yeast one-hybrid system the sequences according to any one of SEQ ID NO 1 to 9 are operably linked to the GAL transcription activator and transformed into yeast cells. These yeast cell are again transformed with a library of constructs encoding candidate regulatory factors.

The present invention will now be described with reference to the following figures in which:
**Figure 1** shows a general schematic representation of a promoter. Regulatory elements are sequences that may for example be responsible for special and/or temporal regulation of the promoter activity. The minimal promoter is the minimal sequence necessary and sufficient to drive expression. It includes a TATA box, which is necessary to correctly direct the RNA polymerase II to the transcription initiation site. The transcription initiation element (INR) includes the transcription initiation start site. The 5' untranslated region (5'UTR) is the region that is transcribed into pre-messenger RNA and eventually into mRNA, but is not translated into protein. The translation initiation codon is represented by the startcodon ATG.
**Figure 2** is a map of the vector p4582 useful for expression in plants of a β-glucuronidase (GUS) gene under control of any one of the promoters according to the invention. This binary vector comprises a Gateway recombination cassette, suitable for the recombination cloning of any of the promoters of the present invention in front of the *Escherichia coli* β-glucuronidase (GUS) gene. This cassette contains a chloramphenicol resistance gene (CamR) and the ccdB suicide gene for counter selection of non-recombined plasmids, This GUS expression cassette further comprises the double terminator sequence T-zein and T-rbcS-deltaGA. This expression cassette is located within the left border (LB repeat, LB Ti C58) and the right border (RB repeat, RB Ti C58) of the nopaline Ti plasmid. Cloned within these borders are also selectable marker and a screenable marker genes each under control of a constitutive promoter and a terminator sequence. This vector also contains an origin of replication (pBR322) for bacterial replication and a bacterial selectable marker (Spe/SmeR) for bacterial selection.
   The following figures show the results of the GUS staining of plants or plant parts transformed with the reporter vector p4582 carrying a promoter according to the present invention operably linked to the reporter gene GUS. Plants denoted "C plants" are transgenic plants grown to about 5 cm; Plants denoted "B plants" are grown to about 10 cm; and plants denoted "A plants" are grown to maturity. These A plants were used to collect different tissue samples from old leaves, young leaves and seeds.

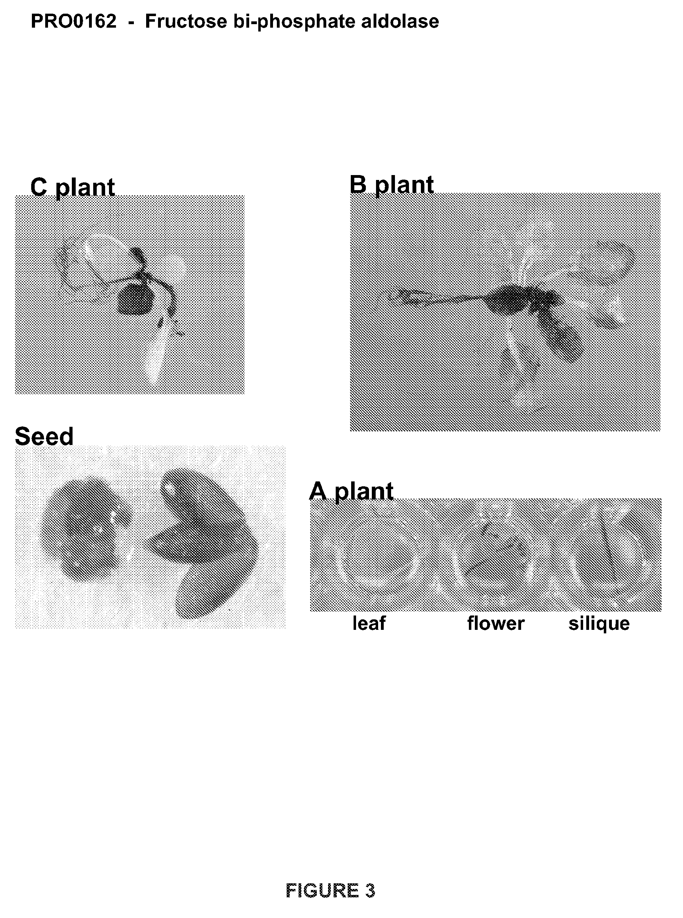

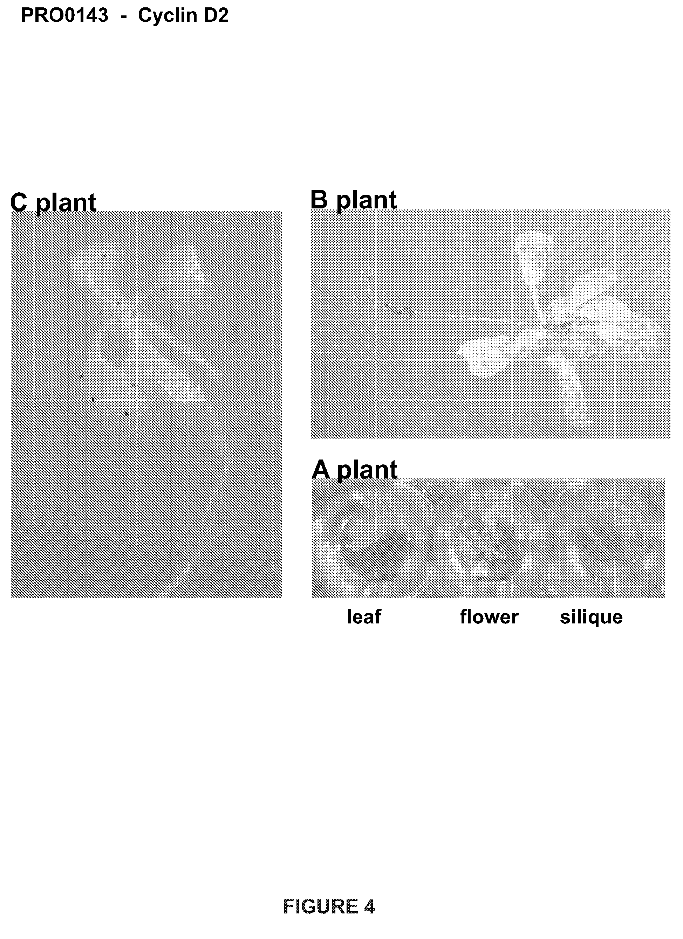

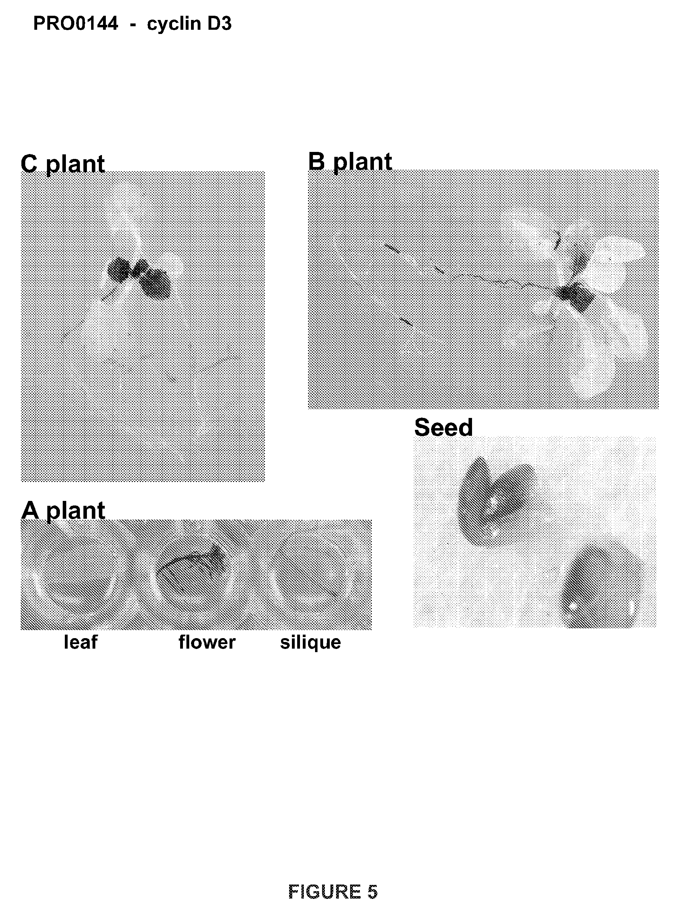

**Figure 3** shows the expression pattern of PRO0162 (fructose bi-phopshate aldolase, SEQ ID NO 1). GUS staining is visible in all parts of the plant.
**Figure 4** shows the expression pattern of PRO0143 (Cyclin D2, SEQ ID NO 2). GUS staining is visible in hydathodes and shoot meristem.
**Figure 5** shows the expression pattern of PRO014 (Cyclin D3, SEQ ID NO 3). GUS staining is visible in actively dividing tissues, root cylinder, hydathodes and seeds.

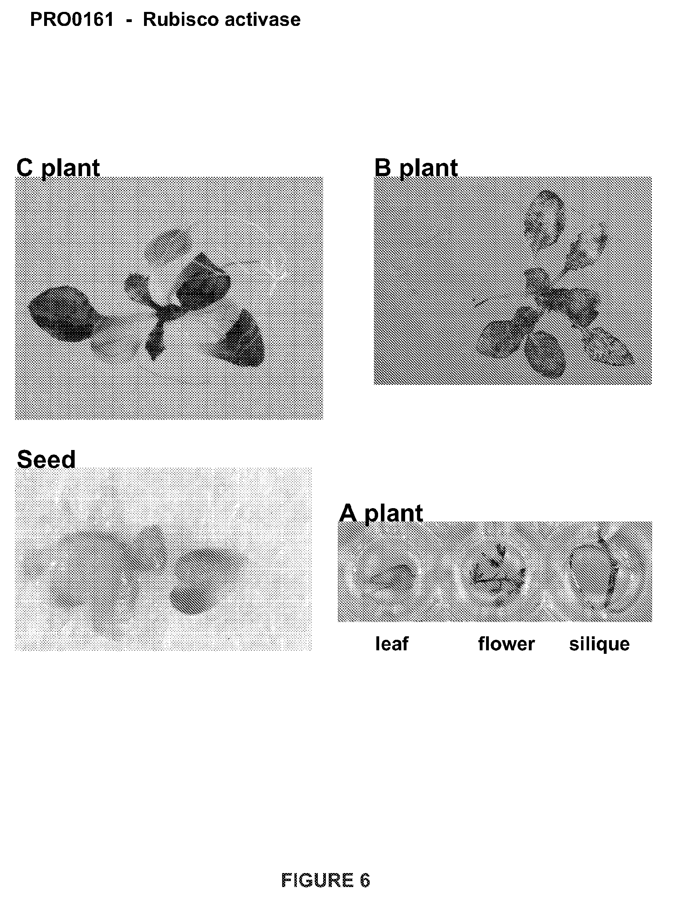

**Figure 6** shows the expression pattern of PRO0161 (rubisco activase, SEQ ID NO 4). GUS staining is visible in shoots, flowers and siliques. GUS staining is also weakly visible in roots and embryos.

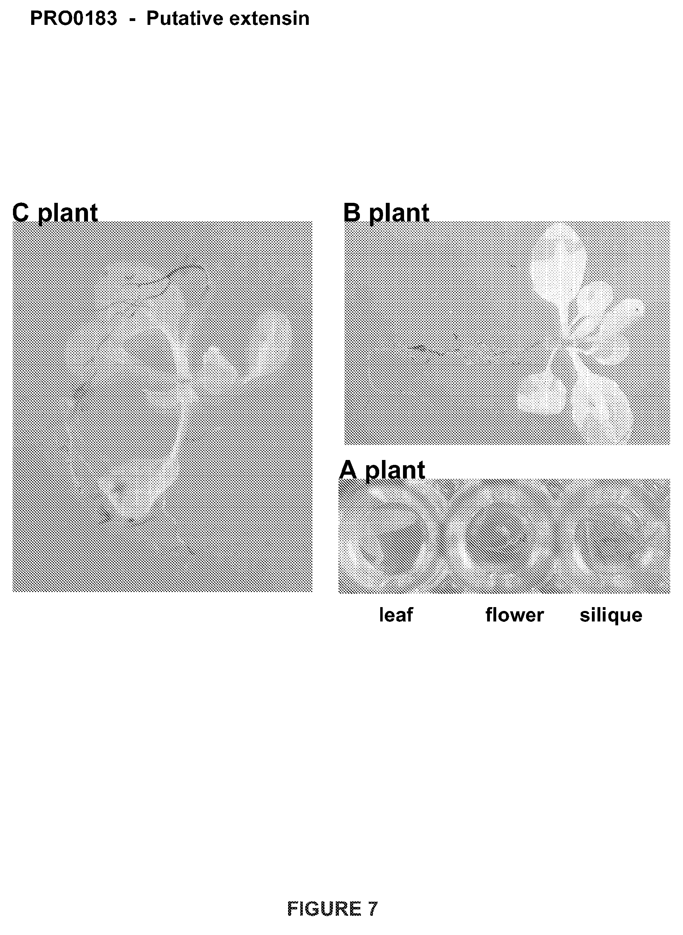

**Figure 7** shows the expression pattern of PRO0183 (putative extensin, SEQ ID NO 5). GUS staining is visible in roots. There is also very weak GUS staining visible in flowers and siliques.

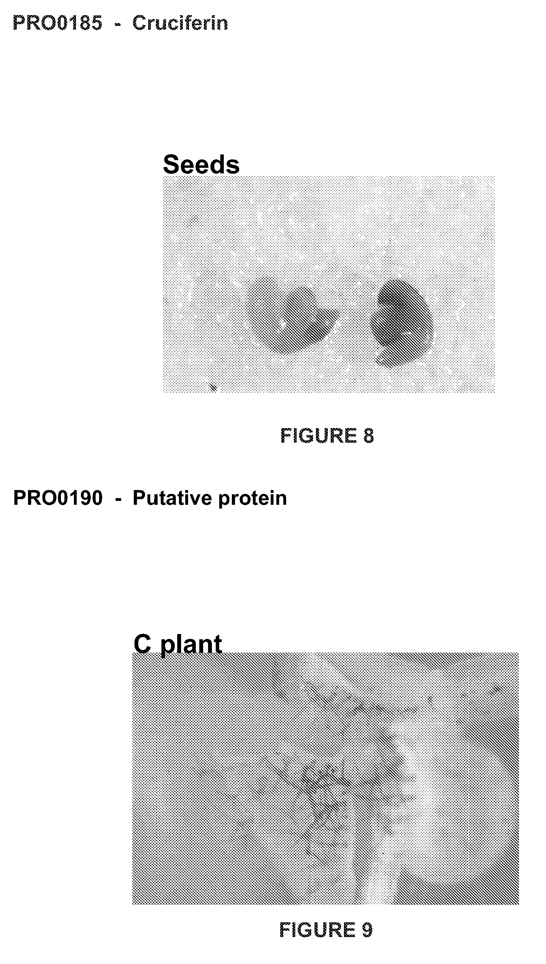

**Figure 8** shows the expression pattern of PRO0185 (12S cruciferin AtCRU3, SEQ ID NO 6). GUS staining is visible in seeds.
**Figure 9** shows the expression pattern of PRO0190 (putative protein, SEQ ID NO 7). GUS staining is visible in trichomes of young developing leaves.

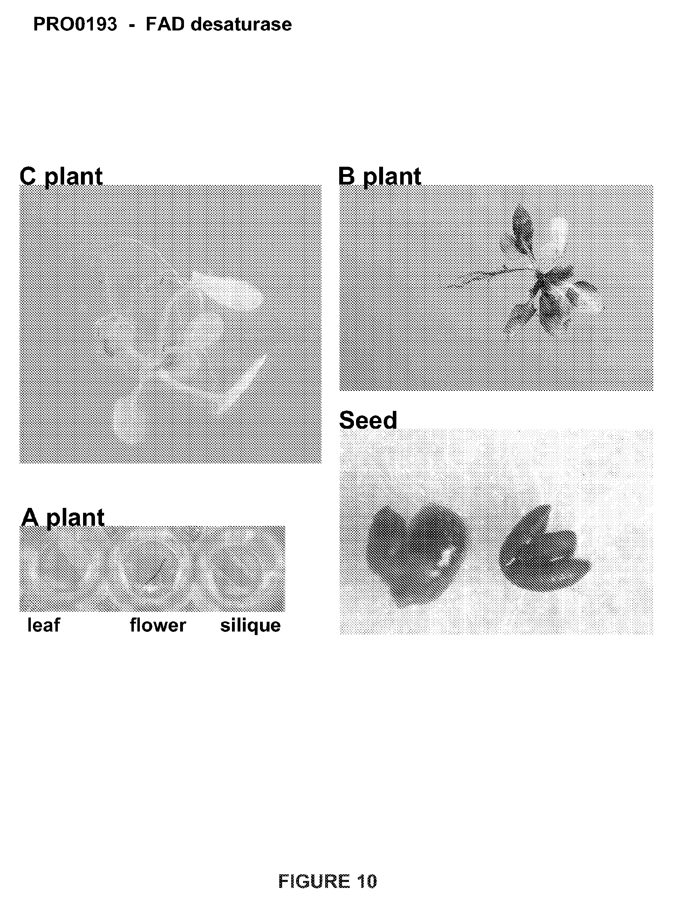

**Figure 10** shows the expression pattern of PRO0193 (FAD2 desaturase, SEQ ID NO 8). GUS staining is visible in young developing tissues, including seeds.

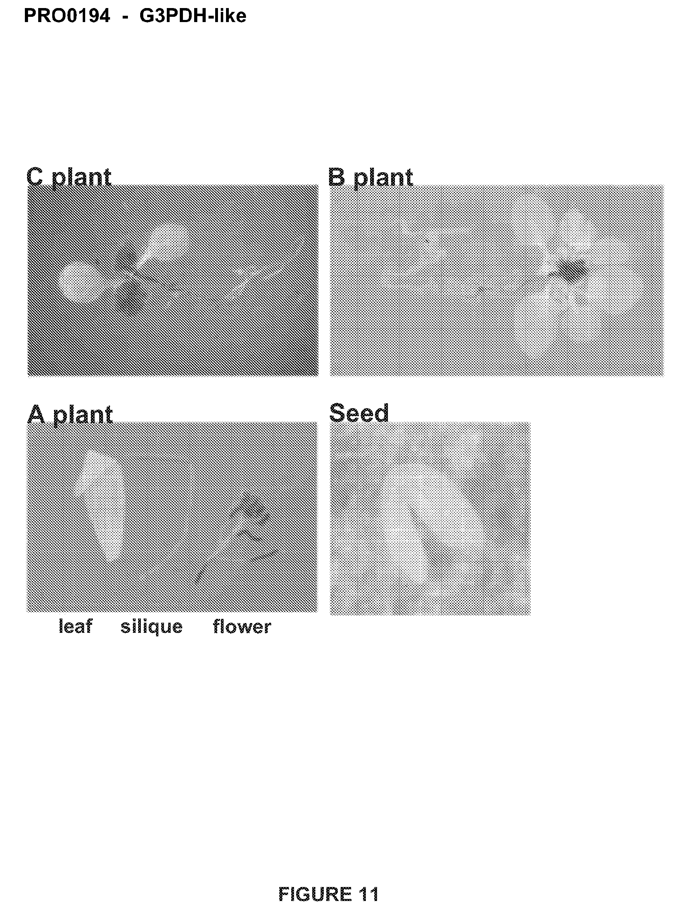

**Figure 11** shows the expression pattern of PRO0194 (G3PDH-like, SEQ ID NO 9). GUS staining is visible in young developing tissues and seeds.

### Examples

The promoters according to the present invention were isolated as DNA regions spanning about 1.2 kb of the sequence upstream of the translation initiation codon (i.e. first ATG, which codon was excluded) from various *Arabidopsis* genes. For determination of their nucleic acid sequence and their expression pattern, the following procedure was followed: First *in silico* studies on genomic *Arabidopsis* sequences were performed. However, procedures based on automated prediction programs to locate promoter-like nucleic acid sequence are highly error prone, even for the localization the best-characterized promoter control elements such as the TATA box and the transcription initiation element (INR). Also, *in silico* determination of expression pattern is extremely speculative. Therefore, to obtain unambiguous data about the nucleic acid sequence and the expression pattern of the promoters, *in vivo* studies were performed encompassing (i) isolation of the promoter nucleic acid sequence; (ii) operably linking a reporter gene to the promoter and introducing the resulting genetic construct into a host organisms; (iii) growing the transformed host cell under conditions allowing expression of the reporter gene; and (iv) determination of the reporter gene activity in the different tissues of the host organism. These methods are now described in more detail.

### Example 1. Identification and isolation of the Arabidopsis promoters

### Identification of ESTs, the corresponding genes and their location in the genome

Sequence databases, comprising *Arabidopsis* sequences, were searched for *Arabidopsis* expressed sequence tags (ESTs). Subsequently an "*in silico*" Northern-blot was performed to allow identification of EST families that are strongly expressed or that are specific for a particular organ. This analysis included normalization of the numbers of ESTs isolated from different plant organs. The ESTs families with an interesting distribution among source cDNA libraries were selected for further analysis and sequence homology searches. After sequence homology searches in combination with scanning scientific data, the genes that correspond to those families of ESTs were identified from sequence databases and a (putative) function and corresponding gene name was given (see Table 1). Subsequently, the corresponding promoter region was isolated by the following procedure. In a first step the TIGR *Arabidopsis* transcribed sequence database was searched to find a tentative contig corresponding to an EST family (http://www.tigr.org/, The Institute for Genomic Research, 9712 Medical Center Drive, Rockville, MD 20850). Sequence homology was found using standard computer programs, such as Blast N using standard parameters (typically G Cost to open a gap = 5, E Cost to extend a gap = 2, q Penalty for a mismatch in the blast portion of run = -3, r Reward for a match in the blast portion of run = 1, e Expectation value = 10.0, W Word size = 11, v Number of one-line descriptions = 100, b Number of alignments to show = 100, Matrix = BLOSUM62). The TIGR database, provides Tentative Contigs (TC) which are sequence predictions based on contig building from all known EST, from all known cDNA and from reconstructed mRNA. The TCs used for identification of the promoters of the present invention are represented by Table 1. In a second step these TCs were used to locate the corresponding gene on a genomic sequence, which gene comprises the coding region as well as the promoter region. Generally, these genomic sequences were BAC clones, which are represented herein by their Genbank accession number (see Table 1). From these BAC clones the sequence identity of the promoter region could be determined.

**Table 1: list of Arabidopsis promoters of the present invention. The promoter sequences are represented herein by their SEQ ID NO and promoter number (PRO). The coding sequences (CDS) naturally driven by a promoter of the present invention are represented by their name, by SEQ ID NO and by Tentative contig (TC) accession number of the TIGR database. The Genomic sequences (BAC clones or genes) comprising a promoter region of the present invention are represented by their Genbank accession number.**

| **Prom SEQ ID NO** | **Prom number** | **CDS name** | **CDS SEQ ID NO** | **CDS TC** | **BAC clone (*or gene)** |
|---|---|---|---|---|---|
| 1 | PR00162 | fructose bi-phopshate aldolase | 10 | TC149436 | AL132969 |
| 2 | PR00143 | Cyclin D2 | 11 | TC156804 | AC006592 |
| 3 | PR00144 | Cyclin D3 | 12 | TC163888 | AL021961 |
| 4 | PR00161 | rubisco activase | 13 | TC160636 + TC160635 | M86720* |
| 5 | PR00183 | putative extensin | 14 | TC160873 +TC160875 | AC004450 |
| 6 | PR00185 | 12S cruciferin AtCRU3 | 15 | TC160506 | AL021749 |
| 7 | PR00190 | putative protein | 16 | TC149672 | AL162651 |
| 8 | PR00193 | FAD2 desaturase | 17 | TC160765 | AP002063 |
| 9 | PR00194 | G3PDH-like | 18 | TC160629 | AC027134 |

### Identification and isolation of the promoter regions of Arabidopsis genes

Starting from the sequence information of the genes and their location in the *Arabidopsis* genome, the promoter regions of these genes were isolated as the DNA region spanning about 1.2 kb upstream of the translation initiation codon (i.e. first ATG), which codon was excluded. When an intervening sequence such as an intron, was present in the 5' untranslated region of the gene, the isolated DNA region was taken as the region spanning about 1.2 kb plus the length of that intervening sequence. The promoter regions were isolated from genomic DNA of *Arabidopsis thaliana* via PCR using specific primers. These specific primers comprise AttB recombination sites, suitable for recombination cloning of the isolated promoter region. These specific primers are herein represented as SEQ ID NO 19 to 36 and are listed in Table 2. Conditions for PCR were as follows: 1 cycle of 2 min at 94°C, 35 cycles of 1 min at 94°C, 1 min at 58°C and 2 min at 68°C, and 1 cycle of 5 min at 68°C. The length of the expected PCR fragment, which is the length of the promoter of the present invention, is also indicated in Table 2. The corresponding PCR fragment was purified from the PCR reaction mix via gel electrophoresis and subsequent purification using Zymoclean Gel DNA Recovery Kit (Zymo Research, Orange, California).

**Table 2: Overview of the primers used to isolate the Arabidopsis promoters of the present invention and the length of the Arabidopsis promoter regions.**

| **Promoter SEQ ID NO** | **Promoter number** | **Prom length** | **Primer forward SEQ ID NO** | **Primer forward** | **Primer reverse SEQ ID NO** | **Primer reverse** |
|---|---|---|---|---|---|---|
| 1 | PR00162 | 1223 | 19 | prm3275 | 28 | prm3276 |
| 2 | PR00143 | 1197 | 20 | prm2943 | 29 | prm2944 |
| 3 | PR00144 | 1219 | 21 | prm2945 | 30 | prm2946 |
| 4 | PR00161 | 1134 | 22 | prm3273 | 31 | prm3274 |
| 5 | PR00183 | 1239 | 23 | prm4475 | 32 | prm4476 |
| 6 | PR00185 | 1067 | 24 | prm4479 | 33 | prm4480 |
| 7 | PR00190 | 1178 | 25 | prm4489 | 34 | prm4490 |
| 8 | PR00193 | 2510 | 26 | prm4498 | 35 | prm4499 |
| 9 | PR00194 | 1050 | 27 | prm4500 | 36 | prm4501 |

### Example 2. Cloning of promoter-GUS reporter vectors for plant transformation

The purified PCR fragments of Example 1, corresponding to the promoter regions of the present invention, were cloned into the pDONR201 entry plasmid of the GatewayTM system (Life Technologies) using the "BP recombination reaction". The identity and base pair composition of the cloned insert was confirmed by sequencing and additionally, the resulting plasmid was tested via restriction digests.

In order to clone each of the promoters of the present invention in front of a reporter gene, each entry clone as mentioned above was subsequently used in an "LR recombination reaction" (Gateway TM) with the destination vector p4582. This destination vector was designed to operably link each promoter of the present invention to the *Escherichia coli* beta-glucuronidase (GUS) gene via the substitution of the Gateway recombination cassette in front of the GUS gene. Furthermore this destination vector is suitable for transformation of plants and comprises within the T-DNA left and right borders the resulting promoter-GUS cassette and selectable marker and screenable marker cassettes (see Figure 2). The resulting reporter vectors, comprising a promoter of the present invention operably linked to GUS, are subsequently transformed into *Agrobacterium* strain LBA4044 and subsequently into *Arabidopsis* plants using standard transformation techniques.

### Example 3. Transformation of Arabidopsis thaliana with promoter-GUS reporter vectors

### Sowing and growing of the parental plants

For the parental *Arabidopsis* plants, approximately 12 mg of wild-type *Arabidopsis thaliana* (ecotype Columbia) seeds were suspended in 27.5 ml of 0.2 % agar solution. The seeds were incubated for 2 to 3 days at a temperature of 4°C and then sown. The plants were germinated under the following standard conditions: 22°C during the day, 18°C at night, 65-70% relative humidity, 12 hours of photoperiod, sub-irrigation with water for 15 min every 2 or 3 days. The seedlings that developed were then transplanted to pots with a diameter of 5.5 cm, containing a mixture of sand and peat in a ratio of 1 to 3. The plants were then further grown under the same standard conditions as mentioned above.

### Agrobacterium growth conditions and preparation

*Agrobacterium* strain C58C1 RIF containing helper plasmid pMP90 and a reporter vector with a promoter of the present invention, was inoculated in a 50 ml plastic tube containing 1 ml LB (Luria Broth) without antibiotic. The culture was shaken for 8-9h at 28°C. Subsequently, 10 ml of LB without antibiotic was added to the plastic tube and shaken overnight at 28°C after which the OD at 600 nm was measured. At an optical density of approximately 2.0, 40 ml of 10% sucrose and 0.05% Silwet L-77 (a chemical mixture of polyalkyleneoxide modified heptamethyltrisiloxane (84%) and allyloxypolyethyleneglycol methyl ether (16%), OSI Specialties Inc) was added to the culture.

### Flower dip

When each parental flowering plant had one inflorescence of 7-10 cm in height, the inflorescences were inverted into the *Agrobacterium* culture and agitated gently for 2-3 seconds. 2 inflorescences per transformation were used. Following this, the plants were returned to the normal growing conditions as described above.

### Seed collection

5 weeks after the flowers were dipped in the Agrobacterium culture, watering of the plants was stopped. The plants were incubated at 25°C with a photoperiod of 20 hours. One week later, the seeds were harvested and placed in a seed drier for one week. The seeds were then cleaned and collected in 15 ml plastic tubes and stored at 4°C until further processing.

### Example 4. Evaluation of the first generation of transgenic Arabidopsis plants comprising the promoter-GUS reporter construct

### Growth and harvest of transgenic plants or plant parts at various stages (C plants, B plants and A plants)

For each transgenic line, seeds were incubated for 2 to 3 days at a temperature of 4°C and then sown. The plants were grown under the following standard conditions: 22°C during the day, 18°C at night, 65-70% relative humidity, 12 hours of photoperiod, sub-irrigation with water for 15 min every 2 or 3 days. At the 2-leaves stage, about 10 plants were sacrificed and stained for GUS expression. These plants are named herein "C plants" for the purpose of evaluation. At the 10-leaves rosette stage, about 10 plants were sacrificed and stained for GUS expression. These plants are named herein "B plants" for the purpose of evaluation. The remaining plants were cultivated until seeds setting. These plants are named herein "A plants" for the purpose of evaluation. At that "A plant" stage, leaves, stems, siliques and seeds were sampled and stained for GUS expression. A plants were thus allowed to set seed, and the remaining seeds were used for confirmation of the expression pattern in plants of the second generation.

### GUS staining

The sacrificed plants or plant parts were covered with 90% ice-cold acetone and incubated for 30 min at 4 °C. After 3 washes of 5 min with Tris buffer [15,76 g Trizma HCI (Sigma T3253) + 2,922 g NaCl in 1 I bidi, adjusted to pH 7,0 with NaOH], the material was covered by a Tris/ferricyanate/X-Gluc solution [9,8 ml Tris buffer + 0,2 ml ferricyanate stock (0,33 g Potassium ferricyanate (Sigma P3667) in 10 ml Tris buffer)+ 0,2 ml X-Gluc stock (26,1 mg X-Gluc (Europa Bioproducts ML 113A) in 500 µl DMSO)]. Vacuum infiltration was applied for 15 to 30 minutes. The plants or plant parts were incubated for up to 16 hours at 37 °C until development of blue colour was visible. The samples were washed 3 times for 5 minutes with Tris buffer. Chlorophyll was extracted in ethanol series of 50%, 70% and 90% (each for 30 minutes).

### Expression patterns of the promoters of the present invention

The expression patterns of the *Arabidopsis* promoters of the present invention are summarized in Table 3.

**Table 3: expression patterns of the Arabidopsis promoters of the present invention**

| **PRO SEQ ID NO** | **Promoter number** | **Promoter name** | **Expression pattern - specificity** |
|---|---|---|---|
| 1 | PR00162 | fructose bi-phopshate aldolase | constitutive |
| 2 | PR00143 | Cyclin D2 | hydathode/meristem |
| 3 | PR00144 | Cyclin D3 | meristem/dividing tissue/seed |
| 4 | PR00161 | rubisco activase | shoot |
| 5 | PR00183 | putative extensin | root |
| 6 | PR00185 | 12S cruciferin AtCRU3 | seed |
| 7 | PR00190 | putative protein | trichomes |
| 8 | PR00193 | FAD2 desaturase | developing tissue preferred |
| 9 | PR00194 | G3PDH-like | developing tissue preferred |

The following paragraphs describe the observed expression patterns of the promoters of the present invention in more detail. The observations are based on the visual inspection of the GUS stained tissues as described above. It is to be understood that for some promoters expression may be weak and that expression in certain tissues may only be visible with very sensitive detection methods.

### PRO0162, SEQ ID NO 1, fructose bi-phopshate aldolase

1 reporter construct (AT1221) was investigated and 8 C, 6 B and 8 A plants were analysed. C plants showed strong expression in the central cylinder of roots (100%), in root tip (75%), in meristem, in hypocotyl (100%) and in all young leaf tissues (100%). C plants showed also weak expression in old hydathodes of leaves (75%) and in cotyledons petioles (37%). B plants showed strong expression in roots (100%), in apical meristem, in hypocotyls (100%), in young leaves (100%). B plants showed also weak expression in old leaves (50%). A plants showed, strong expression in flowers (100%) but weak expression in petals (71 %), strong expression in silique suture (86%) and strong expression in seeds. Therefore, promoter PRO0162 is suitable for expression in all parts of the plant. This promoter is suitable as constitutive promoter and is especially active in young tissues.

### PRO0143, SEQ ID NO 2, Cyclin D2

1 construct (AT1218), which is a reporter vector as described in Example 2 comprising PRO0143 was investigated. 8 C plants, 6 B plants and 7 A plants were analysed. C plants showed strong expression in apical meristem (100%) and in hydathodes of young and old leaves (100%). B plants showed weak expression in hypocotyls (60%), strong expression in apical meristem (100%) and strong expression in hydathodes of young leaves (80%). A plants showed strong expression in stamen (86%). Therefore, promoter PR00143 is suitable for expression especially in hydathodes and meristem, such as shoot meristem.

### PRO0144, SEQ ID NO 3, Cyclin D3

1 construct (AT1220) was investigated and 7 C, 7 B and 8 A plants were analysed. C plants showed strong expression in the central cylinder of roots (86%), strong expression in meristem (86%), strong expression in all young leaves tissues (86%), strong expression in hydathodes of old leaves (86%), but no expression in cotyledons. B plants showed strong expression in the central cylinder of roots (100%) except root tip, strong expression in apical meristem, strong expression in hypocotyls (100%), strong expression in young leaves except trychomes (100%) and expression in hydathodes of old leaves (71%). A plants showed strong expression in pedicel, pistil (100%) and in cotyledons of embryos, but no expression in leaves or siliques, Therefore, promoter PRO014 is suitable for expression in actively dividing tissues, such as meristems, root cylinder, hydathodes and seeds.

### PRO0161, SEQ ID NO 4, rubisco activase

1 construct (AT1222) was investigated and 8 C, 5 B and 6 A plants were analysed. C plants showed strong expression in shoots (75-100%) and no expression in roots. B plants showed strong expression in shoot (100%) and weak expression in roots (40-60%). A plants showed strong expression in flower (100%), expression in siliques and leaves (33%-66%) and weak expression in embryos. It was concluded that PRO016 is highly active in shoot with some leakiness in roots.

### PRO0183, SEQ ID NO 5, putative extensin

1 construct (AT1316) was investigated and 7 C, 6 B and 7 A plants were analysed. C plants showed expression in roots, apical meristem and weak expression in hydatodes. B plants showed expression in the central cylinder of roots, in hypocotyls and apical meristem, but no expression in leaves. A plants showed expression in stamen and siliques, but no expression in leaves and no expression in seeds. Therefore, promoter PRO0183 is suitable as a root-preferred promoter, with some weak expression in flowers and siliques.

### PRO0185, SEQ ID NO 6, 12S cruciferin AtCRU3

1 construct (AT1321) was investigated and 8 C, 7 B and 7 A plants were analysed. C plants showed no expression. B plants showed no expression in leaves and some leaky expression in roots. A plants showed no expression in shoots or leaves, but there was strong expression in seeds. Therefore, promoter PRO0185 is suitable as a seed-specific promoter.

### PR00190, SEQ ID NO 7, putative protein

1 construct (AT1391) was investigated and 8 C, B and 2 A plants were analysed. Expression was observed only in trichomes of C plants. No expression was observed in B plants or A plants. Therefore, promoter PRO0190 is suitable as a promoter specific for trichomes of young developing leaves.

### PRO0193, SEQ ID NO 8, FAD2 desaturase

1 construct (AT1317) was investigated and 8 C, 6 B and 8 A plants were analysed. C plants showed some expression in the central cylinder of roots (25-62%), but not in lateral roots, some expression in apical meristem (62%) and some expression in young leaves (50%). B plants showed strong expression everywhere (66-100 %) except in trychomes and in old leaves, where the expression was rather low. A plants showed strong expression in seeds and some expression in pedicels, stamen (57-71%) and siliques (37-25%). A plants showed no expression in leaves.

Therefore, promoter PRO0193 is suitable for expression in young developing tissues, including seeds. Promoter PRO0193 is considered to be constitutive in young active tissues.

### PRO0194, SEQ ID NO 9, G3PDH-like

1 construct (AT1421) was investigated and 8 C, 7 B and A plants were analysed. C plants showed strong expression in the central cylinder of roots (71 %) and in young leaves (100 %), but no expression in cotyledons. B plants showed also strong expression in the central cylinder of roots (71 %) and in young leaves (100 %), but no expression in lateral roots or old leaves. B plants showed also expression in hydathodes of old leaves. A plants showed expression in flowers and siliques. Therefore, promoter PRO0194 is suitable for expression in young developing tissues, but not in mature tissues and seed.

### Example 5. Stability of the expression patterns of the promoters of the present invention in further generations

The above-mentioned analyses were performed on plants originating from the seeds of transformed flowers. The stability of promoter activity in the next generations or progeny plants of the original T0 plant, the so-called T1 and T2 plants, was evaluated as follows. The T0 plant transformed with the reporter constructs as mentioned in Example 2, were grown until maturity (A plants), of which the seeds (T1 seeds) were harvested and sown to generate progeny T1 plants. These plants were analysed as described above in Example 3 and the A T1 plants were allowed to reach maturity and to set T2 seeds.

The expression pattern of the promoters of the present invention was studied in T0 plants, T1 seeds, T1 plants and T2 seeds and in all the tissues as described in Example 3. The specific expression patterns as reported from the T0 and T1 seeds and described in Example 4 were confirmed in the following T1 generation and T2 seeds. It is concluded that the expression pattern of the promoters of the present invention are stably inherited in plants of subsequent generations.

### Example 6. Stability of expression patterns of the promoters of the present invention in other plants

The above-mentioned plant analyses were performed on *Arabidopsis thaliana* plants. This choice was based on the practical consideration that *Arabidopsis thaliana* plants are good model plants for many dicots and monocots. The reporter constructs comprising the promoters according to the present invention are also transformed into other plants, such as rice or corn, and these transformed plants are evaluated as described hereinabove. The expression patterns of the promoters according to the present invention are conserved among plants. Therefore, the promoters according to the present invention are also suitable for driving and/or regulating expression of an operably linked nucleic acid in monocots, such as rice or corn.

## Claims

1. An isolated promoter capable of driving and/or regulating expression, comprising:
(a) an isolated nucleic acid as represented by any one of SEQ ID NO 9, 1, 2, 3, 4, 5, 7, or 8, or the complement of any one of SEQ ID NO 9, 1, 2, 3, 4, 5, 7, or 8; or
(b) an isolated nucleic acid having at least 90% sequence identity with any one of the DNA sequences as represented by any one of SEQ ID NO 9, 1, 2, 3, 4, 5, 7, or 8; or
(c) an isolated nucleic acid specifically hybridizing under stringent conditions with any one of the DNA sequences as represented by any one of SEQ ID NO 9, 1, 2, 3, 4, 5, 7, or 8; or
(d) an isolated nucleic acid as defined in any one of (a) to (c), which is interrupted by an intervening sequence; or
(e) a fragment of any one of the nucleic acids as defined in (a) to (d), which fragment is capable of driving and/or regulating expression.

2. A promoter according to claim 1, which is a hybrid promoter comprising at least one part of a promoter as defined in claim 1 and further comprising a part of another promoter.

3. A genetic construct comprising:
(a) an isolated promoter as defined in claim 1 or 2; and
(b) a heterologous nucleic acid sequence operably linked to said promoter of (a); and optionally
(c) a 3' transcription terminator.

4. An expression cassette comprising a genetic construct as defined in claim 3.

5. A transformation vector comprising a genetic construct as defined in claim 3.

6. An expression vector comprising a genetic construct as defined in claim 3.

7. A host cell comprising an isolated promoter as defined in claim 1 or 2, or genetic construct as defined in claim 3, or an expression cassette as defined in claim 4, or a transformation vector as defined in claim 5, or an expression vector as defined in claim 6.

8. Host cell according to claim 7, selected from a bacteria, algae, fungi, yeast, plant, insect and animal host cell.

9. A transgenic plant cell comprising an isolated promoter as defined in claim 1 or 2, or a genetic construct as defined in claim 3, or an expression cassette as defined in claim 4 or a transformation vector as defined in claim 5 or an expression vector as defined in claim 6.

10. Transgenic plant cell according to claim 9, which is a monocot plant cell.

11. Transgenic plant cell according to claim 10, which is a dicot plant cell.

12. A transgenic plant comprising a transgenic plant cell as defined in any of claims 9 to 11.

13. A transgenic plant according to claim 12, wherein said plant is selected from rice, maize, wheat, barley, millet, oats, rye, sorghum, soybean, sunflower, canola, sugarcane, alfalfa, bean, pea, flax, lupinus, rapeseed, tobacco, tomato, potato, squash, papaya, poplar and cotton.

14. Plant part, preferably a harvestable part, a propagule or progeny of a plant as defined in claim 12 or 13.

15. Method for driving and/or regulating expression of a nucleic acid in a plant or plant cell, comprising:
(a) operably linking said nucleic acid to a promoter as defined in claim 1 or 2, and
(b) introducing the resultant genetic construct into a plant or plant cell.

16. Method according to claim 15, wherein said expression is constitutive or tissue-specific.

17. Method for the production of a transgenic plant, comprising:
(a) introducing into a plant cell an isolated promoter as defined in claim 1 or 2, or a genetic construct as defined in claim 3, or an expression cassette as defined in claim 4, or a transformation vector as defined in claim 5 or an expression vector as defined in claim 6, and
(b) cultivating said plant cell under conditions promoting plant growth.

18. Use of any of an isolated promoter as defined in claim 1 or 2 to drive and/or regulate expression of an operably linked nucleic acid.
